# EUROPEAN PATENT SPECIFICATION

(11) **EP 1 768 679 B1**
(45) Date of publication and mention of the grant of the patent: **22.08.2012**
(21) Application number: 05758628.1
(22) Date of filing: 08.07.2005
(51) Int. Cl.: A61K 33/14, A61K 31/7105, A61K 31/505, A61P 31/00, A61K 31/497, A61K 31/40, A61K 31/404, A61P 35/00, A61P 35/02, A61K 45/06, A61K 31/704, A61K 33/00, A61K 31/4745

(54) **MODULATION OF GSK-3BETA AND METHOD OF TREATING PROLIFERATIVE DISORDERS**
MODULIERUNG VON GSK-3BETA UND VERFAHREN ZUR BEHANDLUNG VON PROLIFERATIVEN ERKRANKUNGEN
MODULATION DE GSK-3BETA ET PROCEDE DE TRAITEMENT DES MALADIES PROLIFERATIVES

(30) Priority: 09.07.2004 US 586296 P
(43) Date of publication of application: 04.04.2007
(73) Proprietor: Agency for Science, Technology and Research, Singapore 138668 (SG)
(72) Inventor: YU, Qiang, Singapore 597149 (SG)
(74) Representative: Jelsch, Emmanuel Edwin
(86) International application number: PCT/SG2005/000223
(87) International publication number: WO 2006/006939

(56) References cited:
- EP-A1- 1 321 033
- WO-A-01/47533
- WO-A-02/24941
- WO-A-03/084543
- US-A1- 2003 181 439
- FISHMAN P. ET AL: 'An agonist to the A3 adenosine receptor inhibits colon carcinoma frowth in mice via modulation of GSK-3Beta and NFkB' ONCOGENE vol. 23, 2004, pages 2465 - 2471, XP008095144
- LIAO X. ET AL: 'Glycogen synthase kinase-3Beta suppression eliminates tumor necrosis factor-related apoptosis-inducing ligand resistance in prostrate cancer' MOLECULAR CANCER THERAPEUTICS vol. 2, no. 11, 2003, pages 1215 - 1222, XP009055613

### SEQUENCE LISTING

<110> Yu, Qiang
<120> MODULATION OF GSK-3b AND METHOD OF TREATING PROLIFERATIVE DISORDERS
<130> 93231-109
<150> US 60/586,296
   <151> 2004-07-09
<160> 1
<170> PatentIn version 3.3
<210> 1
   <211> 19
   <212> DNA
   <213> artificial
<220>
   <223> synthetic oligonucleotide
<400> 1
   gactccagtg gtaatctac 19

## Description

### FIELD OF THE INVENTION

The present invention relates generally to an agent that inhibits GSK-3β activity for use in the treatment of a cellular proliferative disorder including cancer, and particularly to methods of inducing cell death *in vitro* in a colorectal cancer cell currently undergoing chemotherapy with a DNA damaging agent.

### BACKGROUND OF THE INVENTION

Normally, cells have mechanisms to control growth and proliferation, such that a cell will only divide and grow under certain circumstances and in a controlled manner. Cells also have mechanisms that induce cell death, or apoptosis, when the normal regulatory mechanisms that govern cell growth and proliferation are subverted. However, in some instances, such proliferation control and apoptotic mechanisms breakdown, allowing the cell to proliferate unchecked, potentially leading to a proliferative disorder or disease within an organism. Such proliferative disorders include cancer and other hypercellular lesions such as psoriasis, warts and keloids.

Mutations and deregulation of the cellular protein p53 is often implicated in various cellular proliferative disorders, including many cancers. In a normal, healthy cell, the p53 pathway may be activated by a diverse range of noxious stimuli, resulting in either cell cycle arrest or apoptosis. The process requires an accumulation of p53 protein, which then mediates its physiological effects by transcriptional activation of target genes (Bums, T. F. and El-Deiry, W. S., J Cell Physiol, 181: 231-239, 1999; Gottlieb, T. M. and Oren, M., Semin Cancer Biol, 8: 359-368, 1998; Levine, A. J., Cell, 88: 323-331, 1997; Vousden, K. H. and Lu, X., Nat Rev Cancer, 2: 594-604, 2002). Current evidence suggests that cells undergo cell cycle arrest through transcriptional activation of cyclin-dependent kinase inhibitor p21 (Deng, C., et al. Cell, 82: 675-684, 1995; Chan, T. A., et al. Genes Dev, 14: 1584-1588, 2000; Brugarolas, J., et al. Nature, 377: 552-557, 1995; el-Deiry, W. S., et al. Cell, 75: 817-825, 1993; Bates, S., et al. Oncogene, 17: 1691-1703, 1998) or apoptosis through induction of pro-apoptotic genes such as Puma, Noxa and Bax (Vousden, K. H. and Lu, X., Nat Rev Cancer, 2: 594-604, 2002; Fridman, J. S. and Lowe, Oncogene, 22: 9030-9040, 2003; Kho, P. S., et al. J Biol Chem, 279: 21183-21192, 2004). The precise mechanisms governing the cell fate to p53 response is poorly understood, although it has been proposed that the p53 response ultimately depends on a balance between transcription of p53-targets that favor growth arrest versus apoptosis. Most chemotherapeutic agents induce cell death through activation of the p53 pathway. Therefore, mutations in the p53 gene result in an evasion of apoptotic pathways, leading to more aggressive phenotypes and resistance to chemotherapy.

WO 01/47533 A (ONTARIO CANCER INST; HOEFLICH KLAUS: LUO JUAN: WOODGETT JIM) discloses that the activity of NF-kB is modulated through the effects of GSK-3 on NK-kB activity. Inhibition or down-regulation of GSK-3 results in decreased NF-kB activity. Inappropriate activation of NF-kB has been linked to inflammation and hyperproliferative disorders. Development of modulatory strategies provide a therapeutic tool for the treatment or prevention of various diseases. Methods are also provided for enhanced killing of tumor cells through the sensitization action of GSK-3 inhibition, when administered in coniunction with apoptosis inducing ligands of TNFR1. Transgenic animals defective in GSK-3 function are also provided.

WO 02/24941 A (UNIV DUNDEE; BIONDI RICARDO; FRAME SHEELAGH) provides methods for identifying compounds that are capable of, for example, inhibiting the activity of GSK3 towards phosphate-dependent (primed) substrates to a greater extent than towards non-phosphate-dependent substrates. Mutant GSK3s and other novel polypeptides, polynucleotides and recombinant cells that are useful in such methods are provided. For example, polypeptides useful in modulating the activity of GSK3 are also provided.

The aim of p53-based pharmacology is to translate an understanding of p53 biology to viable therapeutic strategies, hence to promote apoptosis in dysfunctionally proliferating cells, including cancer cells. Genetic approaches that manipulate the level of p53 target genes (Seoane, J., et al. Nature, 419: 729-734, 2002) or p53 transcription co-factors (Iyer, N. G., et al. Proc Natl Acad Sci U S A, 101: 7386-7391, 2004) have been used in different systems to modulate the fine balance in expression levels of p53 targets, consequently promoting p53-dependent apoptosis. While these experiments have been important in understanding the cellular - control of p53-induced cell fate, therapeutic outcomes have yet to arise from these observations.

Recent studies have indicated that p53 can directly induce apoptosis at the mitochondria. p53 has been shown to directly activate pro-apoptotic Bcl-2 family member Bax or Bak (Seoane, J., et al. Nature, 419: 729-734, 2002; Chipuk, J. E., et al. Science, 303: 1010-1014, 2004; Mihara, M., et al. Mol Cell, 11: 577-590, 2003) and forms inhibitory complexes with anti-apoptotic bcl-X_{L} and Bcl2 proteins (Mihara, M., et al. Mol Cell, 11: 577-590, 2003). These interactions eventually result in mitochondria permeabilization and cytochrome c release. This alternative p53 death pathway correlates with an early phase of apoptosis, which occurs independently of p53 transcriptional activity (Erster, S., et al. Mol Cell Biol, 24: 6728-6741, 2004), and can be modulated by direct pharmacological activation of p53 (Erster, S., et al. Mol Cell Biol, 24: 6728-6741, 2004; Chipuk, J. E., et al. Cancer Cell, 4: 371-381, 2003). For the moment, little is know about the regulation of this pathway. Nonetheless, pharmacological activation of this p53-dependent, transcription-independent apoptotic pathway might provide a feasible strategy for therapeutic treatments for proliferative. disorders, including cancer.

### SUMMARY OF THE INVENTION

The majority of chemotherapeutic agents act through an activation of p53 tumor suppressor to induce cell death in cancer cells. However, p53 apoptotic function is often lost in cancer cells due to mutations in p53 or alterations in other components of the p53 pathway, resulting in the resistance to chemotherapy in cancer cells containing such mutations or alterations. Due to suppressed p53 apoptotic function, chemotherapeutic agents will have limited response in these cancer cells. Here, a unique role for glycogen synthesis kinase-3β (GSK-3β) in regulating p53 functions in proliferating cells such as cancer cells, including human colorectal cancer cells, is described. As described herein, pharmacological modulation of GSK-3β can restore p53 apoptotic function in a cell undergoing chemotherapy, thereby promoting apoptosis in such cancer cells, including human colorectal cancer cells undergoing chemotherapy, and further allowing cancer cells, including human colorectal cancer cells, to respond efficiently to chemotherapy.

Thus, in one aspect there is provided an agent that inhibits GSK-3β activity for use in the treatment of a cellular proliferative disorder of a colorectal cancer cell in a subject currently undergoing chemotherapy with a DNA damaging agent, wherein inhibition of GSK-30 activity causes an increase in inhibitory serine phosphorylation of GSK-3β and wherein the colorectal cancer cell is capable of being induced to undergo p53-dependent apoptosis.

In a further aspect, there is provided a method of inducing cell death *in vitro* in a cell currently undergoing chemotherapy with a DNA damaging agent comprising contacting the cell with an agent that inhibits glycogen synthase kinase-3β activity, wherein inhibition of GSK-3β activity causes an increase in inhibitory serine phosphorylation of GSK-3β and wherein the colorectal cancer cell is capable of being induced to undergo p53-dependent apoptosis.

In another aspect there is provided a method of identifying a compound useful for inducing cell death in a cell, comprising:
(a) contacting glycogen synthase kinase-3β with a test compound; and
(b) determining whether inhibitory serine phosphorylation of said glycogen synthase kinase-3β is increased in the presence of said test compound, thereby inhibiting the activity of said glycogen synthase kinase-3β.

### BRIEF DESCRIPTION OF THE DRAWINGS

In the figures, which illustrate, by way of example only, embodiments of the present invention,

**Figure 1A** depicts flow cytometry profiles showing cell cycle distribution of HCT116 and HCT116 p53 -/- cells treated with LY2119301 (5 µM), ADR (1 µM) or both for 24 h. Apoptotic (sub-G1) fractions as indicated;

**Figure 1B** is graphs showing the percentage of apoptosis observed in HCT116 colorectal cells expressing wild type or no p53;

**Figure 2** depicts flow cytometry profiles of HCT116 cells treated as in Figure 1A, and stained for active anti-caspase-3. Percentages of cells positive for active caspase-3 are indicated;

**Figure 3** depicts flow cytometry profiles showing cell cycle distribution of HCT116 and HCT116 p53 -/- cells treated with etoposide (10 µM) or camptothecin (CPT, 100 nM) for 24 h, with or without LY2119301. Apoptotic (subG1) fractions as indicated;

**Figure 4** depicts flow cytometry profiles showing cell cycle distribution of RKO-cells treated with Adriamycin or etoposide, with or without LY2119301, or with LY2119301 alone. Apoptotic (subG1) fractions as indicated;

**Figure 5** depicts dose-response of HCT116 cells to varying 5-FU concentrations, in the presence or absence of LY2119301. Apoptotic fractions determined by flow cytometry;

**Figure 6** depicts analysis for p53 levels in HCT116 cells expressing p53 shRNA or control vector as indicated *(left panel).* Indicated cells were treated with LY2119301 and ADR (1µM or 5-FU (100µM) for 24 h and were subjected to Flow cytometry analysis. Apoptotic (sub-G1) fractions as indicated;

**Figure 7** depicts flow cytometry profiles of RKO and RKO/E6 cells treated as in Figure 1A. Apoptotic (sub-G1) fractions as indicated; .

**Figure 8** depicts chemical structures of three GSK-3 inhibitors: LY2119301, SB-216763 and SB-415286;

**Figure 9** depicts flow cytometry profiles of HCT116 cells treated with lithium and DNA damage as before. Apoptotic (sub-G1) fractions as indicated;

**Figure 10** depicts flow cytometry profiles showing cell cycle distribution of HCT116 cells treated with ADR and SB-216763 or SB-415286. Apoptotic (sub-G1) fractions as indicated;

**Figure 11** depicts an immunoblot showing relative levels of p53, p21, PARP and β-actin in cells treated with LY21193D1, SB-216763 and SB-415286, with or without ADR;

**Figure 12** depicts immunoblot analysis for phospho-ser9/21-GSK3α/β, phosphor-tyr216/279-GSK3α/β and total GSK-3α/β in HCT116 cells treated with lithium, LY2119301, Sub-216763 or SB-415286;

**Figure 13** depicts HCT116 cells were transfected with GSK-3β or GSK-3a specific siRNA and negative control siRNA (NC siRNA) before treated with 10 uM etoposide for 24h. Levels of GSK-3α/β and PARP cleavage were analyzed by immunoblotting;

**Figure 14** depicts immunoblot analysis for phospho-ser9/21-GSK3α/β, phosphor-tyr216/279-GSK3α/β and total GSK-3α/β in cells treated with LY2119301, SB-216763 or SB-415286, with or without ADR in nuclear and cytoplasmic fractions;

**Figure 15** depicts immunoblot analysis for p53, p21, puma, Bax and PARP in HCT116 cells treated with ADR (*upper panel*), etoposide (*middle panel*), *5-*FU (*bottom panel*), in the presence or absence of LY2119301 for the indicated times and doses. β- Actin is shown as a loading control;

**Figure 16** depicts immunoblot analysis for p53, p21, puma, Bax and PARP in RKO cells treated as in Figure 15;

**Figure 17** depicts immunoblot analysis for p53, p21, puma, Bax and PARP in HCT116 cells; treated with the indicated drugs, with or without lithium (*left panel*); and treated with ADR in the presence of LY119301, SB-216673 or SB-415286 (*right panel*);

**Figure 18** depicts microarray analysis showing p53-responsive genes in **HCT116** treated with ADR alone or with ADR and LY2119301. Cluster and Tree Viewer were used to cluster 12 selected genes that have been previously identified as p53 targets in HCT116 cells;

**Figure 19** depicts p53-inducible DLD1 cells were cultured in medium with (tet off) or without (tet on) 20 ng/ml doxycycline for 24 h. Left panel: Flow cytometry profiles of DLD1 cells in the presence or absence of LY2119301. Right panel: Western blot showing levels of p53, p21 and PARP;

**Figure 20** depicts HCT116 cells infected with Ad-p53 or Ad-lacZ as indicated were subjected to LY2119301 or DMSO treatment for 24 h. Cells were subjected to flow cytometry analysis (*left panel*) and immunoblot analysis for p53, p21 and PARP (*right panel*). NS- loading control;

**Figure 21** depicts flow cytometry profiles showing cell cycle distribution of HCT116 Bax -/- cells treated with ADR or etoposide in the presence or absence of LY2119301 for 24 h. Apoptotic (sub-G1) fractions as indicated;

**Figure 22** depicts flow cytometry profile of HCT116 Puma -/-cells as in Figure 21;

**Figure 23** depicts immunoblot analysis for p53, p21 and PARP in HCT116 Bax -/-cells, treated as indicated;

**Figure 24** depicts immunoblot analysis for p53, p21 and PARP in HCT116 Puma -/- cells, treated as indicated;

**Figure 25** depicts western blots showing Bax activation in HCT116 cells treated with indicated drugs for 24 h (*upper panel*). Western blots of cytosolic cell fractions showing Cytochrome c release and the proteolysis of caspase-9 in HCT1 16 co-treated with LY2119301 and ADR or etoposide (*lower panel*);

Figure 26 depicts IP-Western blots showing Bax activation in DLD1 cells with p53 induction (*left panel*) and HCT116 cells with p53 over-expression (r*ight panel*), and treated with LY2119301;

Figure 27 depicts flow cytometry profile showing JC-1 staining as an indicator of mitochondrial membrane potential in HCT116 and HCT116 Bax -/- cells. Percentage of cells with ψₘ loss is indicated; and

Figure 28 depicts time-course analysis of p53 accumulation in the cytosol and mitochondria following etoposide and 5-FU treatments (upper panel). p53 accumulation in the presence or absence of LY2119301 following etoposide treatment for 24 h. Levels of p53 and mitochondria marker CoxIV were determined by Western blot analysis.

### DETAILED DESCRIPTION

The present invention described herein relates to an agent that inhibits GSK-3β activity for use in the treatment of a cellular proliferative disorder of a colorectal cancer cell in a subject currently undergoing chemotherapy with a DNA damaging agent, wherein inhibition of GSK-3β activity causes an increase in inhibitory serine phosphorylation of GSK-3β and wherein the colorectal cancer cell is capable of being induced to undergo p53-dependent apoptosis.

Glycogen synthase-3 kinase (GSK-3) is a constitutively expressed serine-tyrosine kinase which has two isoforms (α and β), and is involved in diverse signaling pathways (Cohen, P. and Frame, S., Nat Rev Mol Cell Biol, 2: 769-776, 2001). Initially described as a key enzyme involved in glycogen metabolism, GSK-3β is now known to regulate a diverse array of cell functions, which functions include a proapoptotic role in neurons and other tissues (Watcharasit, P., et al., J Biol Chem, 278: 48872-48879, 2003; Hetman, M., et al., J Neurosci, 20: 2567-2574, 2000; Bijur, G. N., et al., J Biol Chem, 275: 7583-7590, 2000).

The within use and methods are based on the findings described herein that pharmacological modulation of GSK-3β markedly impairs p53-dependent transactivation of targets including p21 and Puma, but promotes p53-dependent conformational activation of Bax, resulting in cytochrome *c* release, loss of mitochondrial membrane potential and caspase-9 processing. These observations indicate that p53-mediated damage response can be converted from cell cycle arrest to apoptosis, particularly following exposure to a variety of chemotherapeutic agents. It was discovered that this effect appears to be associated with the modulation of inhibitory serine 9 phosphorylation of GSK-3β but not with the activating tyrosine phosphorylation. It was further found that the induction of apoptosis appears to occur through a direct mitochondrial pathway that requires Bax but not Puma.

In the findings described herein, GSK-3β is observed to participate in regulating p53 activity in both the nucleus and mitochondria. In contrast to other studies which demonstrate a pro-apoptotic role for GSK-3β, the present methods and uses are based on the observation that pharmacological modulation of GSK-3β dramatically promotes p53-induced apoptosis in proliferating cells undergoing chemotherapy, including cancer cells such as colorectal cancer cells. Apoptosis occurs even with concurrent impairment of p53 transcriptional activity, and appears to be mediated through direct mitochondrial p53 activity, which appears to require Bax but not Puma. Without being bound to any particular theory, constitutively expressed cellular kinase GSK-3β appears to have dual but distinct roles in regulating p53 pathways: promoting transcriptional activity of p53 in the nucleus but suppressing direct apoptotic activity of p53 at the mitochondria.

Thus, the present invention relates to an agent that inhibits GSK-3β activity for use in the treatment of uncontrolled proliferating cells undergoing chemotherapy as defined in the claims. Such inhibition results in promotion, induction or enhancement of the apoptotic response in such cells, allowing for treatment of proliferative disorders.

In one embodiment, there is provided a method of inducing cell death *in vitro* in a colorectal cancer cell currently undergoing chemotherapy with a DNA damaging agent comprising contacting the cell with an agent that inhibits Glycogen synthase kinase-3β activity, wherein inhibition of GSK-3β activity causes an increase in inhibitory serine phosphorylation of GSK-3β and wherein the colorectal cancer cell is capable of being induced to undergo p53-dependent apoptosis.

Glycogen synthase kinase ("GSK-3β") as used herein refers to the β isoform of glycogen synthase kinase-3, which plays a role in cellular apoptotic response and includes mammalian GSK-3β, including human GSK-3β. The term includes homologs, fragments, derivatives or variants of GSK-3β that possess the apoptotic regulatory activity of GSK-3β, which activity can be modulated so as to induce an apoptotic response in a proliferating cell.

A polynucleotide sequence or polypeptide sequence is a "homolog" of, or is "homologous" to another sequence if the two sequences have substantial identity over a specified region and the functional activity of the sequences is conserved (as used herein, the term "homologous" does not imply evolutionary relatedness). Two polynucleotide sequences or polypeptide sequences are considered to have substantial identity if, when optimally aligned (with gaps permitted), they share at least approximately 50% sequence identity, or if the sequences share defined functional motifs. In alternative embodiments, optimally aligned sequences may be considered to be substantially identical (i.e. to have substantial identity) if they share at least 60%, 70%, 75%, 80%, 85%, 90%, 95%, 96%, 97%, 98%, 99% identity over a specified region. An "unrelated" or "non-homologous" sequence shares less than 40% identity, and possibly less than approximately 35%, 30% or 25% identity, with a polypeptide or polynucleotide of the invention over a specified region of homology. The terms "identity" and "identical" refer to sequence similarity between two peptides or two polynucleotide molecules. Identity can be determined by comparing each position in the aligned sequences. A degree of identity between amino acid sequences is a function of the number of identical or matching amino acids at positions shared by the sequences, i.e. over a specified region. Optimal alignment of sequences for comparisons of identity may be conducted using a variety of algorithms, as are known in the art, including the Clustal W program, available at http://clustalw.genome.ad.jp, the local homology algorithm of Smith and Waterman, 1981, Adv. Appl. Math 2: 482, the homology alignment algorithm of Needleman and Wunsch, 1970, J. Mol. Biol. 48:443, the search for similarity method of Pearson and Lipman, 1988, Proc. Natl. Acad Sci. USA 85: 2444, and the computerised implementations of these algorithms (such as GAP, BESTFIT, FASTA and TFASTA in the Wisconsin Genetics Software Package, Genetics Computer Group, Madison, WI, U.S.A.). Sequence identity may also be determined using the BLAST algorithm, described in Altschul et al., 1990, J. Mol. Biol. 215:403-10 (using the published default settings). Software for performing BLAST analysis is available through the National Center for Biotechnology Information (through the internet at http//www.ncbi.nlm.nih.gov/). As used herein, "homologous amino acid sequence" includes any polypeptide which is encoded, in whole or in part, by a nucleic acid sequence which hybridizes at 25-35°C below critical melting temperature (Tm), to any portion of a nucleic acid sequence encoding mammalian GSK-3β, including human GSK-3β.

A variant or derivative of GSK-3β refers to a GSK-3β protein or a fragment thereof, which retains the apoptotic regulatory activity of GSK-3β, or a GSK-3β that has been mutated at one or more amino acids, including point, insertion or deletion mutations, but still retains the apoptotic regulatory activity of GSK-3β. A variant or derivative therefore includes deletions, including truncations and fragments; insertions and additions, for example conservative substitutions, site-directed mutants and allelic variants; and modifications, including peptoids having one or more nonamino acyl groups (q.v., sugar, lipid, etc.) covalently linked to the peptide and post-translational modifications. As used herein, the term "conserved amino acid substitutions" or "conservative substitutions" refers to the substitution of one amino acid for another at a given location in the peptide, where the substitution can be made without substantial loss of the relevant function. In making such changes, substitutions of like amino acid residues can be made on the basis of relative similarity of side-chain substituents, for example, their size, charge, hydrophobicity, hydrophilicity, and the like, and such substitutions may be assayed for their effect on the function of the peptide by routine testing.

The "apoptotic regulatory activity" or "apoptotic activity" of GSK-3β refers to the ability of GSK-3β to interact with or influence the p53 apoptotic pathways, including the p53 transcriptional activation pathway in the nucleus and the direct effect of p53 induced apoptosis in the mitochondria. The apoptotic regulatory activity of GSK-3β is effected at least in part by inhibitory serine phosphorylation of GSK-3β. For example, increased phosphorylation at Ser9 in human GSK-3β results in an increased apoptotic response of the direct mitochondrial p53 apoptotic pathway. The apoptotic regulatory activity of GSK-3β may also include the ability of GSK-3β to promote p53-activated transcription.

Inducing or induction of cell death means to initiate cell death or apoptosis or to enhance the ability or tendency of a cell to undergo cell death or apoptosis, or to enhance the sensitivity of a cell to events that may trigger cell death or apoptosis, and as used herein refers to such inducing as a result, directly or indirectly, of modulation of GSK-3β activity. Cell death or apoptosis can be measured using standard apoptosis assays or by detecting known apoptotic markers, for example, cytochrome c release, loss of mitochondrial membrane potential, caspase3 activation, caspase-9 processing, PARP cleavage or increased sub-G1 population in a cell culture or cell population, as will be understood in the art. Inducing cell death may be performed *in vitro* or *in vivo.*

When referring to a cell in which cell death is to be induced, or a cell that is a proliferating cell, including a cell that is involved in a proliferative disorder, the term "cell" refers to a single cell, a plurality of cells or a population of cells, unless otherwise indicated herein. The cell may be any cell in which proliferation or division is desired to be inhibited, or in which cell death is desired to be induced, including a cell that has lost the ability to undergo cell cycle arrest or apoptosis. The cell may be a cell in culture or it may be a cell within a patient. As used herein, proliferation of a cell refers to the process of DNA replication, growth and division, which leads to an increase in the total number of cells. The cell may be derived from any organism that expresses a GSK-3β homolog, and in particular embodiments is a mammalian cell, including a mouse cell, a rat cell, a rabbit cell or a human cell.

The cell may be a cell that is currently undergoing chemotherapy, meaning that the cell is currently being treated with a chemotherapy regimen, including simultaneously with, overlapping with, or sequentially to the inhibition of GSK-3β activity, provided that the benefit or effect of the chemotherapy treatment is ongoing in the cell concomitantly with the inhibition. Thus, "currently undergoing chemotherapy" includes chemotherapy that covers a greater or lesser period of time as the time period for which the activity of GSK-3β is to be inhibited. Chemotherapy treatments and chemotherapeutic agent are known in the art, and are further described herein.

Cell death is induced in a cell by inhibition of GSK-3β activity in such a cell. "Inhibiting" or "inhibition of" the apoptotic regulatory activity of GSK-3β refers to any mechanism of, deregulating, , disrupting, interrupting, reducing, limiting, blocking or preventing the ability of GSK-3β to perform its biological function or activity, including regulating, inducing, effecting or interacting with p53-dependent apoptotic pathways, thereby resulting in induction of cell death. Inhibition includes the effect of enhancing or inducing p53-dependent apoptosis, or inhibiting or down-regulating p53-activated transcription. Inhibition includes physical alteration of GSK-3β, for example by post-translational modification, loss or lack of necessary post-translational modification, mutation of the amino acid sequence, including deletion, insertion and substitution mutation, or protein digestion. Inhibition also includes stabilizing, inducing, antagonizing, inhibiting, or competing with, the interaction between GSK-3β and apoptotic factors which are targets of GSK-3β, and which GSK-3β normally interacts with to effect cell cycle progression or inhibition of cell death. Such post-translational modification includes inhibitory phosphorylation, for example, serine phosphorylation that reduces the kinase activity of GSK-3β, as well as inhibition or down-regulation of activating phosphorylation, for example reduced levels of tyrosine phosphorylation of GSK-3β. Inhibition further includes pharmacological inhibition, for example, antagonism, competitive or non-competitive inhibition with an inhibitor of GSk-3β, for example a small molecule inhibitor or a protein or peptide inhibitor.

Thus, the cell is treated so as to inhibit the activity of GSK-3β in the cell. Inhibition may be accomplished by treating the cell with an agent that inhibits the activity of GSK-3β. The agent may be an inhibitor of GSK-3β activity, for example, a compound that inhibits, interferes with, competes with or interrupts the apoptotic regulatory activity of GSK-3β, including a molecule that causes, stimulates or enhances inhibitory serine phosphorylation of GSK-3β or a molecule that inhibits or down-regulates activating tyrosine phosphorylation of GSK-3β.

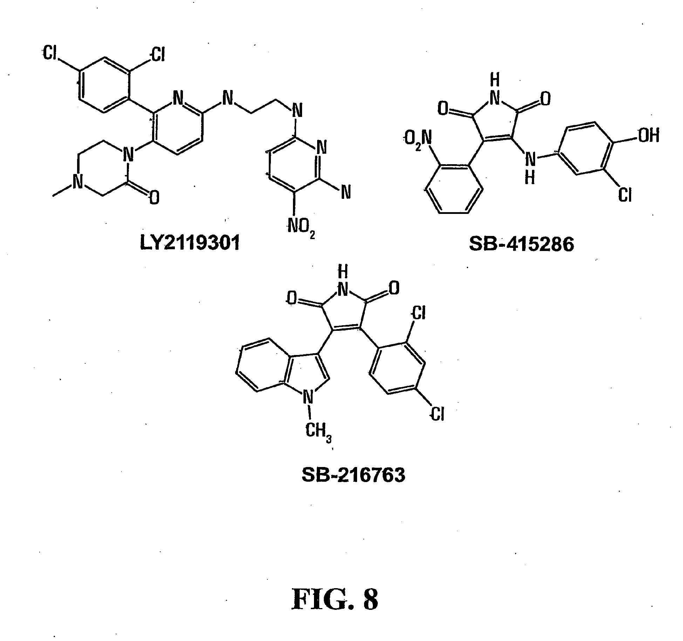

In a particular embodiment, the agent is the small molecule LY2119301, the chemical structure of which is depicted in **Figure 8**. In another embodiment, the agent is lithium-containing compound, including lithium chloride.

The agent may be capable of being delivered internally to a cell, for example by active or passive transport into the cell, or by diffusion into the cell. For example, if the agent is a small molecule, it may be soluble in the cell membrane and thus able to permeate the cell. The agent may also be modified to include a transport tag that will facilitate its transport into a cell. Specific transport tags may be used in order to direct the agent to be taken up by specific target cells. For example, the agent may be modified to include a galactose residue to increase uptake of the agent by hepatocytes, as is described in US 6,844,319. Where the agent is a peptide, inclusion of a sequence such as a membranetranslocating sequence that allows the peptide in which it is included to be transported into a cell, for example the penetratin sequence derived from the *Drosophila melanogaster* antennapedia homeodomain protein, facilitates the uptake of the agent by the cell. Alternatively, the agent may be included in a biomaterial which increases or induces uptake of the agent by the cell, for example, by encapsulating the agent in a liposome preparation. Liposome delivery of peptides and proteins to cells is known, and is described for example in US 6,372,720 and US 20030108597.

Thus, inhibition of GSK-3β apoptotic regulatory activity may be achieved by exposure of the cell to the agent, allowing for uptake of the agent by the cell, allowing the agent to interact with GSK-3β, a target of GSK-3β, or otherwise interact with the p53-dependent apoptosis pathways so as to inhibit the activity of GSK-3β.

The inhibition may be further accomplished in combination with a chemotherapeutic agent. "In combination" with a chemotherapeutic agent means that the inhibition occurs in a time period during which a chemotherapeutic agent is contacted with or administered to the cell. The inhibition of GSK-3β activity and the administration of the chemotherapeutic agent may occur simultaneously or sequential, and the respective time period for each may be conterminous or may be overlapping. The inhibition and the administration each may be achieved in one or more discrete treatments or may be performed continuously for a given time period required in order to achieve the desired result.

The cell may be further treated with the chemotherapeutic agent in a manner similar to that described above for treatment with the agent that inhibits GSK-3β activity, depending on the nature of the chemotherapeutic agent. The cell may be treated with the chemotherapeutic agent prior to, following, or simultaneously with the agent that that modulates GSK-3p activity.

The chemotherapeutic agent may be a compound that is typically administered to a cell and which has a cytotoxic or cytostatic effect. The chemotherapeutic agent may be an agent that induces apoptosis, such as p53-dependent apoptosis, or that induces cell cycle arrest, including p53-dependent cell cycle arrest, in a cell that is abnormally proliferating, even in the absence of an agent that inhibits GSK-3β activity. The chemotherapeutic may also be an agent that activates p53 or p21 in an abnormally proliferating cell but that does not induce apoptosis in the cell, due to a property of the abnormally proliferating cell, for example an alteration or mutation in p53 or in the p53 pathways. Treatment of the cell with the chemotherapeutic agent in combination with the agent that inhibits GSK-3β activity induces cell death, or increases sensitivity to cell death, at a level greater than that which is observed in the absence of the agent that inhibits GSK-3β activity.

The chemotherapeutic agent may be a DNA damaging agent or a genotoxic agent that can activate p53-dependent apoptosis or p53-dependent cell cycle arrest in a proliferating cell. The chemotherapeutic agent may be, without limitation, a small molecule, a peptide or a protein, an anthracycline, an alkylating agent, an alkyl sulfonate, an aziridine, an ethylenimine, a methylmelamine, a nitrogen mustard, a nitrosourea, an antibiotic, an antimetabolite, a folic acid analogue, a purine analogue, a pyrimidine analogue, an enzyme, a podophyllotoxin, a platinum-containing agent or a cytokine. Preferably, the chemotherapeutic agent is one that is known to be effective against the particular cellular proliferative disorder and cell type. In certain embodiments the chemotherapeutic agent is Adriamycin (ADR), 5-fluorouracil (5-FU), etoposide, or camptothecin or a derivative or analog thereof.

Inhibition of GSK-3β activity is useful in vitro and in vivo, and may be used in the treatment or inhibition of diseases or disorders that relate to abnormally proliferating cells, in which it is desirable to induce cell death. Preferably the treatment comprises inducing cell death in the colorectal cancer cell and preferably the subject is a human.

A "proliferative disorder" is a disease or disorder in which a cell of a patient is abnormally proliferating, resulting in uncontrolled growth and division of the cell, which in a healthy individual would not be proliferating or would be proliferating in a controlled manner. The proliferative disorder may be characterized by the proliferation of malignant or non-malignant cell populations. Such disorders include cancer including colorectal cancer, bladder cancer, colon cancer, prostate cancer, lung cancer, nasopharyngeal carcinoma, cervical carcinoma, skin cancer, leukemia; hyperplastic skin lesions including genital warts, psoriasis and keloids; ischemic heart disease; acquired immune deficiency syndrome; vasculitis; rheumatoid arthritis; athersclerosis; glomerulonephritis leading to glomerulosclerosis; interstitial inflammation leading to fibrosis; pulmonary inflammation leading to fibrosis. A proliferating disorder includes abnormal proliferation which may also occur during normal wound healing, potentially leading to excessive scarring or keloid formation, and which may occur during any inflammatory condition leading to tissue injury in which host immune cells trigger fibroblasts to proliferate and elaborate extracellular matrix components leading to fibrosis. Examples of the latter include the host immune response to cerebral infarction (stroke), myocardial infarction (heart attack) and acute ischemic renal tubular injury (transient kidney failure). In a particular embodiment the proliferative disorder is colorectal cancer.

A cell is associated with a proliferative disorder if that cell is a cell that is abnormally proliferating so as to result in the disorder in the patient, or if the disorder is characterized by the proliferation of such a cell.

The term "treating" a proliferative disorder refers to an approach for obtaining beneficial or desired results, including clinical results. Beneficial or desired clinical results can include, but are not limited to, alleviation or amelioration of one or more symptoms or conditions, diminishment of extent of disease, stabilization of the state of disease, prevention of development of disease, prevention of spread of disease, delay or slowing of disease progression, delay or slowing of disease onset, amelioration or palliation of the disease state, and remission (whether partial or total). "Treating" can also mean prolonging survival of a patient beyond that expected in the absence of treatment. "Treating" can also mean inhibiting the progression of disease, slowing the progression of disease temporarily, although more preferably, it involves halting the progression of the disease permanently.

The subject is any animal in need of treatment of a proliferative disorder, including a mammal, including a human, and including a subject that is currently undergoing chemotherapy.

The therapeutic effect is achieved by administering to the patient an agent that modulates the activity of GSK-3β, including a molecule that modulates the apoptotic regulatory activity of GSK-3β. The agent that modulates the activity of GSK-3β is any agent that may be used to effect modulation in a cell as described above, including an agent that increases, enhances or promotes inhibitory serine phosphorylation of GSK-3β.

An effective amount of the agent that inhibits the activity of GSK-3β is administered to the patient. The term "effective amount" as used herein means an amount effective, at dosages and for periods of time necessary to achieve the desired result, for example, to treat the specific proliferative disorder.

The agent is administered to the patient using standard techniques known in the art. The agent may be administered systemically, or may be administered directly at the site at which the proliferating cell that is associated with the proliferative disorder is located. Delivery to the site includes topical administration, injection to the site, or surgical implantation, for example at a site of a tumour.

The concentration and amount of the agent that inhibits the activity of GSK-3β to be administered will vary, depending on the proliferative disorder to be treated, the type of cell associated with the proliferative disorder, the type of molecule that is administered, the mode of administration, and the age and health of the patient.

The agent that inhibits the activity of GSK-3β may be administered in combination with a chemotherapeutic agent as described above. A "combination" of the agent that modulates the activity of GSK-3β and a chemotherapeutic agent for administration may be formulated together in the same dosage form or may be formulated in separate dosage forms, and the separate dosage forms may be the same form or different forms, for administration by the same mode or by different modes of administration. Furthermore, administration of a combination of the agent that inhibits the activity of GSK-3β and a chemotherapeutic agent, when not together in the same dosage form, means that the agent that inhibits the activity of GSK-3β and a chemotherapeutic agent are administered concurrently to the subject being treated, and may be administered at the same time or sequentially in any order or at different points in time. Thus, the agent that inhibits the activity of GSK-3β and a chemotherapeutic agent may be administered separately but sufficiently closely in time so as to provide the desired therapeutic effect.

The agent that inhibits the activity of GSK-3β, optionally in combination with a chemotherapeutic agent, may be administered as a sole therapy or may be administered in combination with other therapies, including radiation therapy or other anti-viral therapies. For example, the agent that inhibits the activity of GSK-3β, optionally in combination with a chemotherapeutic agent, may be administered either prior to or following surgical removal of a primary tumour or prior to, concurrently with or following treatment such as administration of radiotherapy.

To aid in administration, the agent that inhibits the activity of GSK-3β may be formulated as an ingredient in a pharmaceutical composition, optionally with a therapeutic agent, including a chemotherapeutic agent, as described above. Therefore, in a further embodiment, there is provided a pharmaceutical composition comprising an agent that inhibits the activity of GSK-3β, and a pharmaceutically acceptable diluent. The invention in one aspect therefore also includes such pharmaceutical compositions for use in treating a proliferative disorder. The compositions may routinely contain pharmaceutically acceptable concentrations of salt, buffering agents, preservatives and various compatible carriers. For all forms of delivery, the agent that inhibits the activity of GSK-3β may be formulated in a physiological salt solution. Since peptides may be unstable upon administration, where the agent that inhibits the activity of GSK-3β is a peptide or a protein, it may be desirable to include the peptide or the protein in a liposome or other biomaterial useful for protecting and/or preserving the peptide or protein until it is delivered to the target cell.

The pharmaceutical compositions may additionally contain other therapeutic agents useful for treating the particular proliferative disorder, for example a cytotoxic agent, for example a chemotherapeutic agent.

The proportion and identity of the pharmaceutically acceptable diluent is determined by chosen route of administration, compatibility with live cells, and standard pharmaceutical practice. Generally, the pharmaceutical composition will be formulated with components that will not significantly impair the biological properties of the agent that inhibits the activity of GSK-3β.

The pharmaceutical composition can be prepared by known methods for the preparation of pharmaceutically acceptable compositions suitable for administration to patients, such that an effective quantity of the agent that inhibits the activity of GSK-3β, and any additional active substance or substances, is combined in a mixture with a pharmaceutically acceptable vehicle. Suitable vehicles are described, for example, in Remington's Pharmaceutical Sciences (Remington's Pharmaceutical Sciences, Mack Publishing Company, Easton, Pa., USA 1985). On this basis, the pharmaceutical compositions include, albeit not exclusively, solutions of the agent that inhibits the activity of GSK-3β, in association with one or more pharmaceutically acceptable vehicles or diluents, and contained in buffer solutions with a suitable pH and iso-osmotic with physiological fluids.

The pharmaceutical composition may be administered to a patient in a variety of forms depending on the selected route of administration, as will be understood by those skilled in the art. The composition of the invention may be administered topically, surgically or by injection to the desired site.

Solutions of the agent that inhibits the activity of GSK-3β may be prepared in a physiologically suitable buffer. Under ordinary conditions of storage and use, these preparations contain a preservative to prevent the growth of microorganisms, and that will maintain the function of the agent that inhibits the activity of GSK-3β. A person skilled in the art would know how to prepare suitable formulations. Conventional procedures and ingredients for the selection and preparation of suitable formulations are described, for example, in Remington's Pharmaceutical Sciences and in The United States Pharmacopeia: The National Formulary (USP 24 NF19) published in 1999.

In different embodiments, the composition is administered topically or by injection (subcutaneously, intravenously, intramuscularly, etc.) directly at the desired site where the cells that are proliferating in an uncontrolled manner are located in the patient.

The dose of the pharmaceutical composition that is to be used depends on the particular condition being treated, the severity of the condition, the individual patient parameters including age, physical condition, size and weight, the duration of the treatment, the nature of concurrent therapy (if any), the specific route of administration and other similar factors that are within the knowledge and expertise of the health practitioner. These factors are known to those of skill in the art and can be addressed with minimal routine experimentation.

In a manner consistent with the above-described use, there is also presently contemplated a method of inducing cell death *in vitro* in a colorectal cancer cell currently undergoing chemotherapy with a DNA damaging agent comprising contacting the cell with an agent that inhibits glycogen synthase kinase-3β activity, wherein inhibition of GSK-3β activity causes an increase in inhibitory serine phosphorylation of GSK-3β and wherein the colorectal cancer cell is capable of being induced to undergo p53-dependent apoptosis. Preferably said inhibiting induces p53-dependent apoptosis and the chemotherapy induces p53-dependent cell cycle arrest. More preferably said inhibiting inhibits p53-activated transcription. In a preferred embodiment of the invention the chemotherapy involves Adriamycin, 5-fluorouracil, etoposide or camptothecin. In particular, the agent that inhibits GSK-3β activity is a lithium-containing compound such as lithium chloride or LY2119301.
Also provided is a method for identifying or screening for agents or compounds useful for inducing cell death in a cell, comprising:
(b) contacting glycogen synthase kinase-3β with a test compound; and,
(b) determining whether inhibitory serine phosphorylation of said glycogen synthase kinase-3β is increased in the presence of said test compound, thereby inhibiting the activity of said Glycogen synthase kinase-3β.
Preferably, step (a) comprises contacting a cell expressing said glycogen synthase kinase-3β with said test compound. Test molecules are exposed to or contacted with GSK-3β or to a biochemical pathway that includes, is regulated by or interacts with GSK-3β, including in the context of a cell expressing GSK-3β, including in a cell undergoing chemotherapy or in combination with a chemotherapeutic agent. Once the test compound is contacted with GSK-3β, the activity of GSK-3β is measured, including the ability of GSK-3β to regulate apoptosis in the cell. The activity of GSK-3β may be measured using known assays, including kinase assays, apoptosis assays, as well examining the phosphorylation state and levels of phosphorylation at inhibitory serine phosphorylation sites within GSK-β.

The methods of screening for agents or compounds that are useful for inducing cell death in a cell are well suited for screening combinatorial libraries of compounds. Thus, the above-noted methods and assays may be employed either with a single test compound or a plurality or library of test compounds. In the latter case, synergistic effects provided by combinations of compounds may also be identified and characterized. In certain embodiments, one or a plurality of the steps of the screening/testing methods of the invention may be automated. Such assay systems may comprise a variety of means to enable and optimize useful assay conditions. Such means may include but are not limited to: suitable buffer solutions, for example, for the control of pH and ionic strength and to provide any necessary components for GSK-β activity and stability (e.g. protease inhibitors), temperature control means for optimal GSK-3β activity and or stability, and detection means to enable the detection of the GSK-3β activity. A variety of such detection means may be used, including but not limited to one or a combination of the following: radiolabelling (e.g. ³²P), antibody-based detection, fluorescence, chemiluminescence, spectroscopic methods (e.g. generation of a product with altered spectroscopic properties), various reporter enzymes or proteins (e.g. horseradish peroxidase, green fluorescent protein), specific binding reagents (e.g. biotin/streptavidin), and others.

All technical and scientific terms used herein have the same meaning as commonly understood by one of ordinary skill in the art of this invention, unless defmed otherwise.

### EXAMPLES

***Example 1***

**Materials and Methods**

**Cell culture and drug treatments:** Human colon cancer cell line HCT116 and its derived isogenic p53 ^{-/-}, Puma^{-/-}, Bax^{-/-} and p53-inducible DLD cell lines were kindly provided by Dr. Bert Vogelstein (Johns Hopkins University, MD). RKO and RKO/E6 cells were from ATCC. Cells were cultured in DMEM containing 10% FBS. All culture reagents and media were from Invitrogen. 5- Fluorouracil, Adriamycin, etoposide and lithium chloride were purchased from Sigma. GSK-3β specific small molecule inhibitor LY2119301 was a gift from Eli Lilly (Indianapolis) and SB-216763, SB-415283 were obtained from BIOMOL.

Western blotting, immunoprecipitation and microarray analysis: Cells were scraped, collected and lysed as described previously (Yu, Q., et al. Cancer Res, 62: 5743-5748, 2002). Briefly, cells were lysed with cell lysis buffer (0.3% NP40, 1 mM EDTA, 50 mM Tris-HCl (pH 7.4), 2 mM EGTA, 1% Triton X-100, 150 mM NaCl, 25 mM NaF, 1 mM Na₃VO₄, 2 mM AEBSF, 5 µg/ml aprotinin, 1 µg/ml leupeptin) for 30 minutes on ice, and the lysates were clarified by centrifugation at 12,000 X g for 15 minutes at 4°C. Protein concentration was quantified (Bio-Rad, Hercules, CA) and protein samples (50 µg) were separated by SDS/PAGE and transferred onto Immobilon^{™} membranes (Millipore, Bedford, MA). Antibodies against the following proteins were used: p53 (DO-1), p21, PARP, GSK-α/ß, α-Tubulin, and ß-actin (Santa Cruz Biotechnology), Puma (Ab-1, Oncogene), Bax (Sigma), phosphor-(Ser9/21)-GSK-3α/ß and Caspase-9 (Cell Signaling Technologies), phosphor-(Tyr216/279)-GSK-3α/ß (Upstate Biotechnologies), cytochrome c (BD PharMingen). To detect Bax conformational change, cells were lysed in 1% Chaps buffer and the soluble fraction was immunoprecipitated with anti-Bax monoclonal antibody (6A7, BD, PharMingen), followed by immunoblotting with anti-Bax polyclonal antibody. Microarray analysis was performed as described (Kho, P. S., et al. J Biol Chem, 279: 21183-21192, 2004).

FACS analysis of DNA content and caspase-3 activity: Cells were harvested and fixed in 70% ethanol. Fixed cells were stained with propidium iodide (50µg/ml) after treatment with RNase (100 µg/ml). The stained cells were analyzed for DNA content by fluorescence-activated cell sorting (FACS) in a FACScalibur^{™} (Becton Dickinson Instrument, San Jose, CA). Cell cycle fractions were quantified using the CellQuest^{™} software (Becton Dickinson). To measure caspase-3 activity, cells were fixed with Cytofix/Cytoperm^{™} solution (BD PharMingen) as instructed, and then stained with FITC-conjugated rabbit anti-active caspase-3 monoclonal antibody (BD PharMingen). Quantification of cells positive for the caspase-3 detection was performed by flow cytometry.

**RNA interference:** To generate knockdown vectors for p53, the following 19-nucleotide sequence was used: gactccagtggtaatctac [SEQ ID NO:1]. The oligonucleotide pair was cloned into the retroviral expression vector pSIREN-RetroQ^{™} (BD Bioscienses) according to the manufacturer's instructions. Briefly, 293 cells were transfected with the p53 shRNA construct for 48 h to produce viral particles. To establish p53 shRNA stable transfection clones, HCT116 cells were infected with retroviral stocks in the presence of 5 µg/ml polybrene. Infected cells were subject to puromycin selection at 2µg/ml. Stable clones were further expanded and screened by Western blot analysis for p53 expression. *SMART*pool® GSK-3β and GSK-3α siRNAs and negative control siRNA were purchased from Dharmacon, Inc.

**Adenoviral infections:** Adenovirtis Ad-LacZ and Ad-p53 were obtained from Dr. Shibin Zhou (Johns Hopkins University, MD). Cells were grown to 50% confluence and infected with recombinant adenovirus as described (Yu, J., et al. Proc Natl Acad Sci U S A, 96: 14517-14522, 1999) . Twenty-four hours after the infection, cells were treated with drugs for the indicated times.

**Subcellular fractionation:** Cells were lysed in mitochondria lysis buffer (210 mM Mannitol, 70 mM Sucrose, 10 mM Hepes (pH, 7.4), 1mM EDTA, protease inhibitor cocktail) with a Dounce homogenizer and subjected to centrifugation at 1000 X g to pellet the nuclei. Post-nuclear supernatant was centrifuged at 10,000 X g to pellet the mitochondria-enriched heavy membrane fraction, and the resulting supernatant was further centrifuged to obtain cytosolic fraction. Total proteins (30 µg) from cytosolic or mitochondrial fractions were subjected to immunoblot analysis.

**Measurement of mitochondrial permeability transition:** Cells were harvested by centrifugation at 300 X g at 4°C for 5 min. Mitochondrial permeability transition was determined by staining the cells with JC-1 (BD PharMingen) according to the manufacturer's instructions. Mitochondrial permeability transition was quantified by flow cytometric determination of cells with decreased red fluorescence, i.e. with mitochondria displaying a lower membrane potential (Δψₘ). Data are given in percentage of cells with low Δψ_{m.}

**Results**

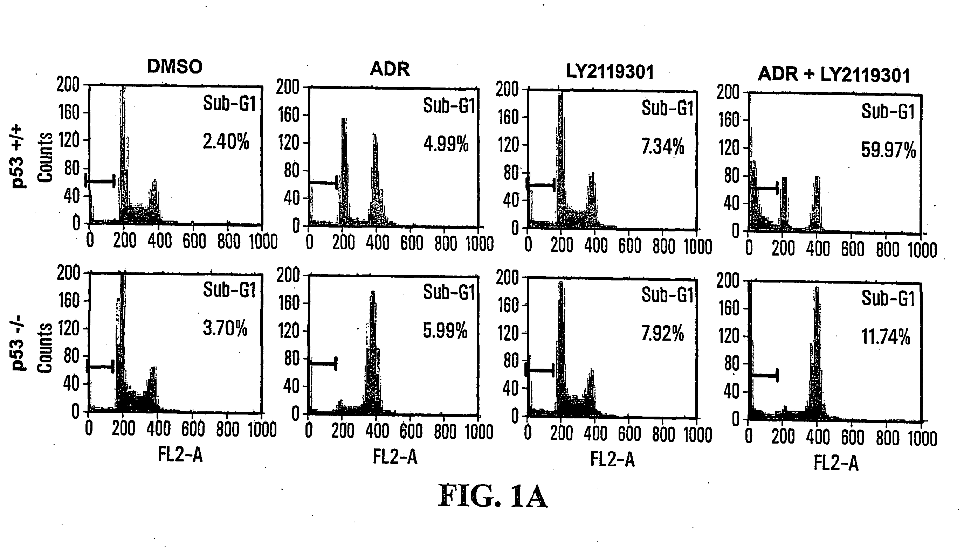

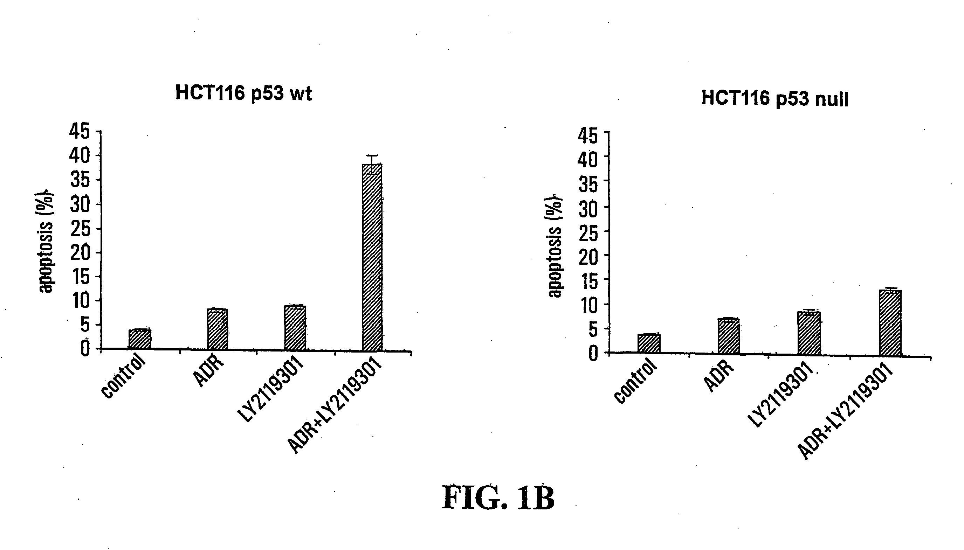

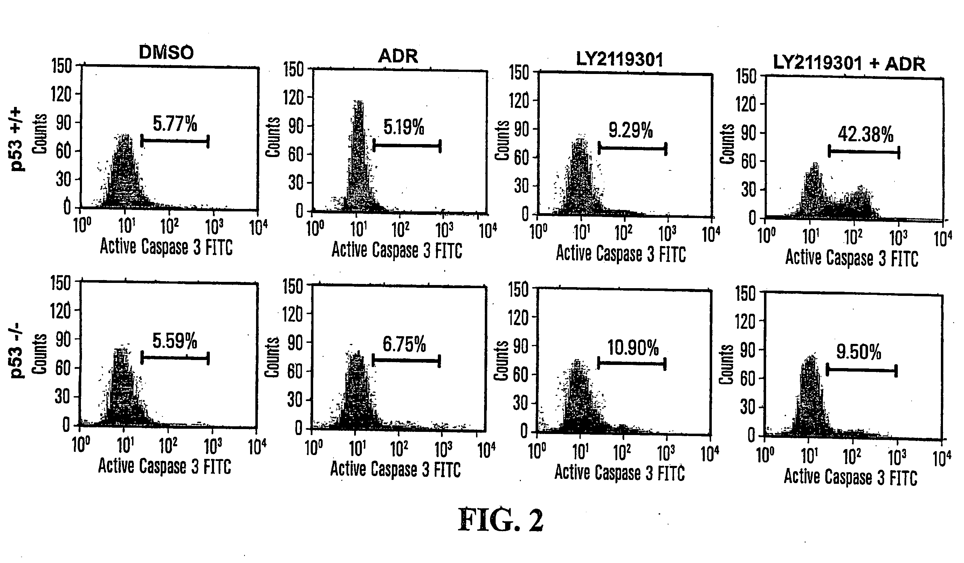

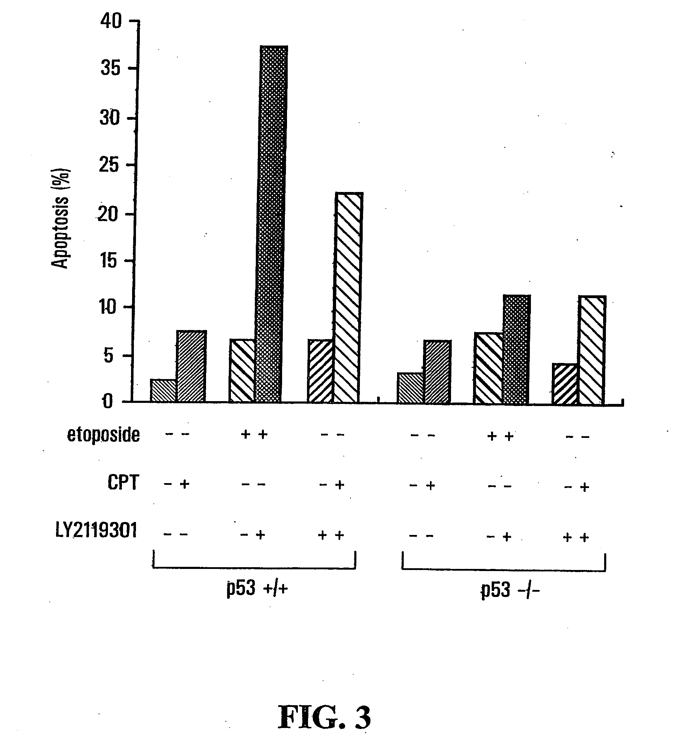

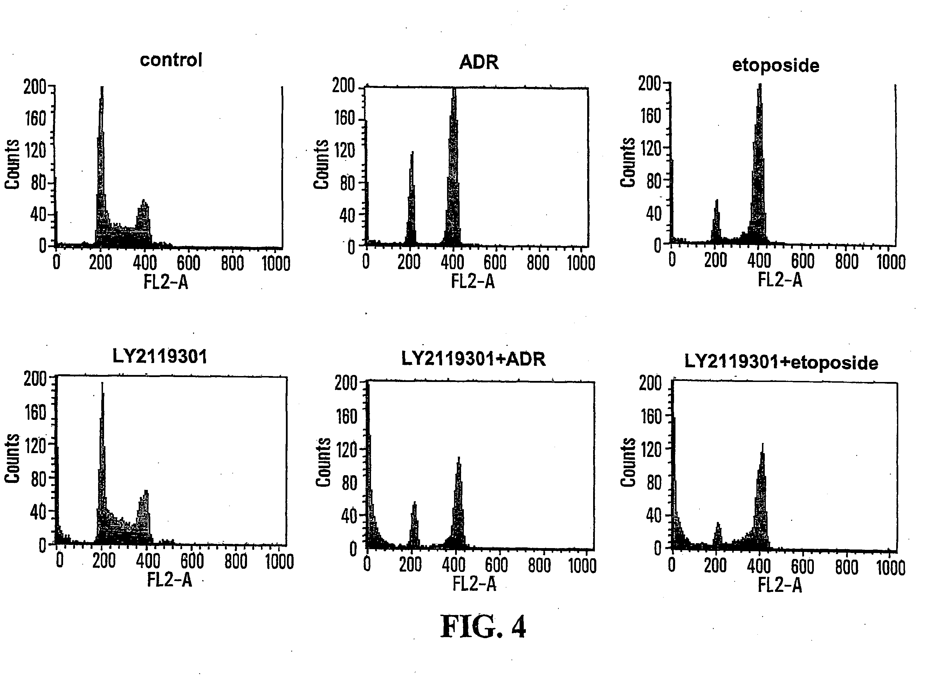

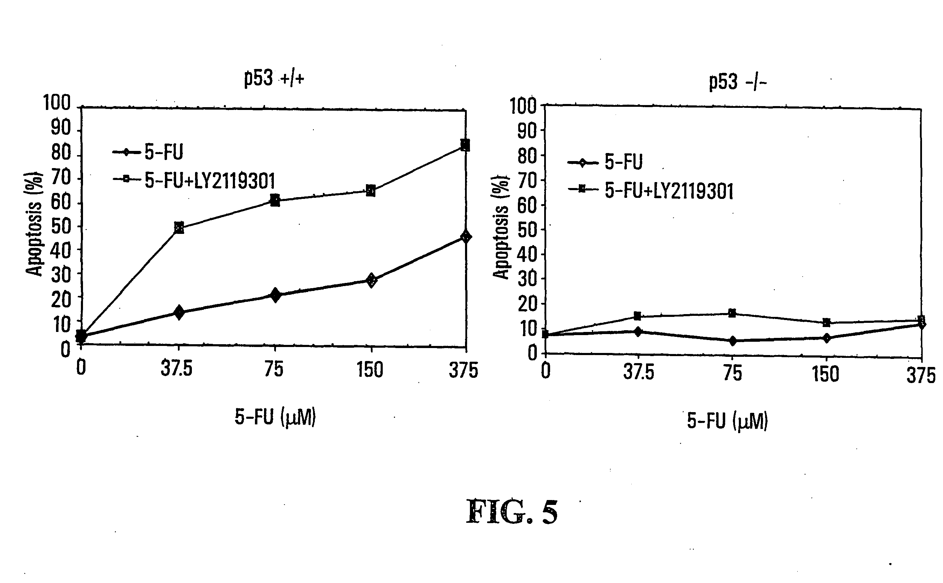

**Small molecule GSK-3β inhibitor promotes genotoxic agent-induced apoptosis in human colorectal cancer cells in a p53-dependent manner:** In human colorectal cancer HCT116 cells, p53 activation by DNA damaging agent Adriamycin (ADR) and DNA analogue 5-Fluororacil (5-FU) results in cell cycle arrest and apoptosis, respectively (Bunz, F., et al. J Clin Invest, 104: 263-269, 1999). To investigate the cellular factors that modulate p53 response, we screened a series of protein kinase inhibitors and examined their effect on DNA damage response. We found that a specific GSK-3β small molecular inhibitor (LY2119301) efficiently converted ADR-induced p53 response from growth arrest to cell death. **Figures 1A** and **1B** show that HCT116 cells treated with ADR for 24 h underwent cell cycle arrest (arrested in both G1 and G2/M phase) without obvious induction of cell death. Co-treatment of cells with ADR and LY2119301 resulted in a marked increase in cell death as evidenced by the sub-G1 population (~ 59%), compared to cells treated with ADR or LY2119301 alone (~3~5%). In contrast, the synergistic induction of cell death was not seen in p53 knockout HCT116 cells (**Figure 1A** and **1B**) under the same conditions, suggesting that the cell death is p53-dependent. The cell death was attributable to apoptosis since similar results were obtained by using a caspase-3 activity assay (**Figure 2**). LY2119301 also triggered p53-dependent apoptosis in response to other DNA damaging agents including etoposide and camptothecin (CPT) (**Figure 3**). Similar results were also observed in another p53 wildtype colon cancer cell line, RKO, but not in p53 mutant tumors such as SW420 and HT29 (**Figure 4** and data not shown). Moreover, LY2119301 greatly sensitized 5-FU-induced apoptosis in a strictly p53-dependent manner (**Figure 5**), as no increased apoptosis was observed in HCT116 cells that are null for p53. Remarkably, dose-effect experiments showed that LY2119301 induced a 10-fold increase in apoptosis induction by 5-FU, which is one of the most commonly used chemotherapeutic agents in the treatment of colorectal cancer. Thus, pharmacological inhibition of GSK-3β not only converted p53-mediated damage response from growth arrest to apoptosis, but also further sensitized p53-dependent apoptotic response. In both cases, inactivation of GSK-3β triggers or sensitizes an apoptotic process that requires p53.

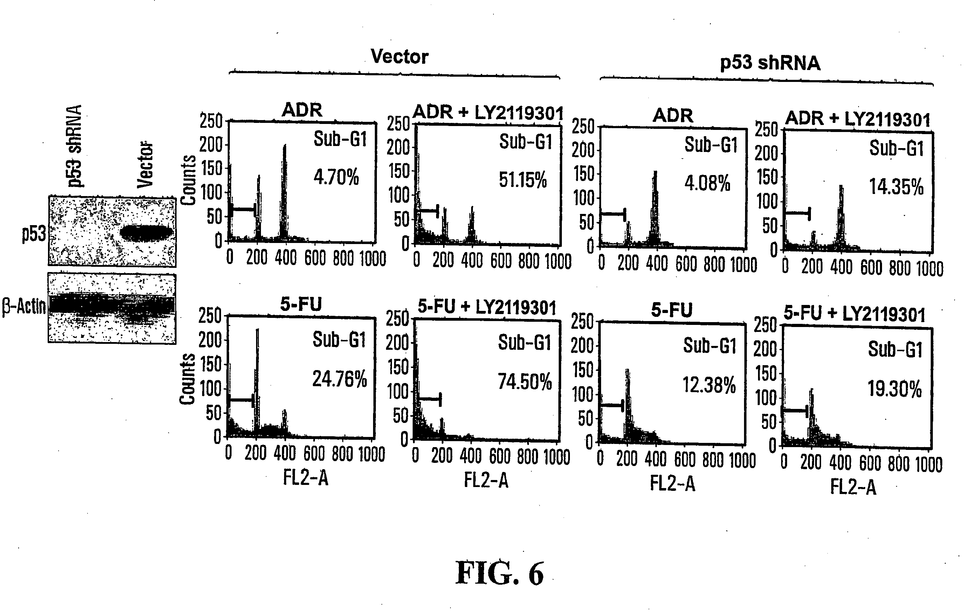

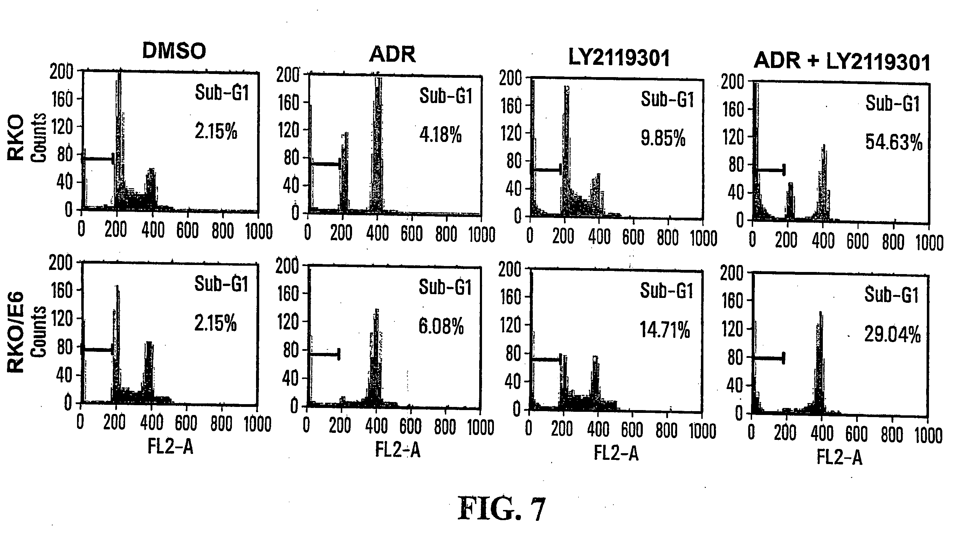

To confirm that the observed increase in apoptosis was truly p53-dependent and is not due to phenotypic traits as a result of prolonged cultures of HCT116 p53 ^{-/-}cells, we generated a stable HCT116 cell line in which p53 expression was greatly silenced by p53 shRNA (**Figure 6****, left panel**). p53 knockdown in these cells significantly reduced the apoptosis induction by co-treatment with LY2119301 and ADR or 5FU, compared to the cells expressing control vector (**Figure 6****, right panel**). The apoptotic effect of LY2119301 on the p53 response was also observed in another p53 wild-type colorectal cancer cell line RKO, but not in its p53-inactive counterpart RKO/E6 cells (**Figure 7**). Similarly, apoptosis was not induced in other p53 mutant colon cancer cell lines SW480 and HT29 (data not shown). These findings suggest that GSK-3β inhibitor LY2119301 promotes apoptosis in a manner that strictly requires intact p53.

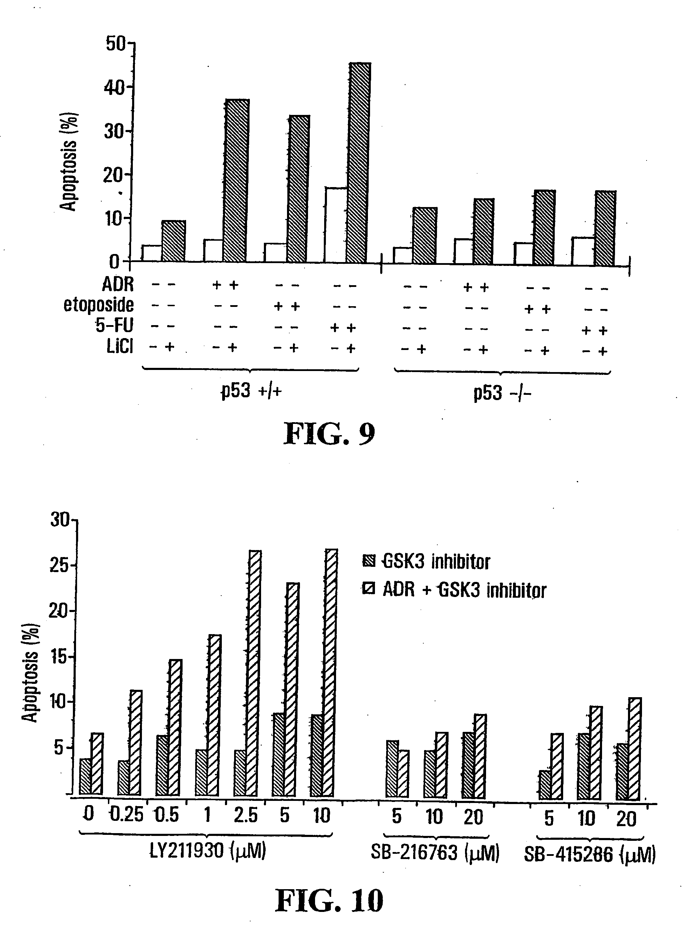

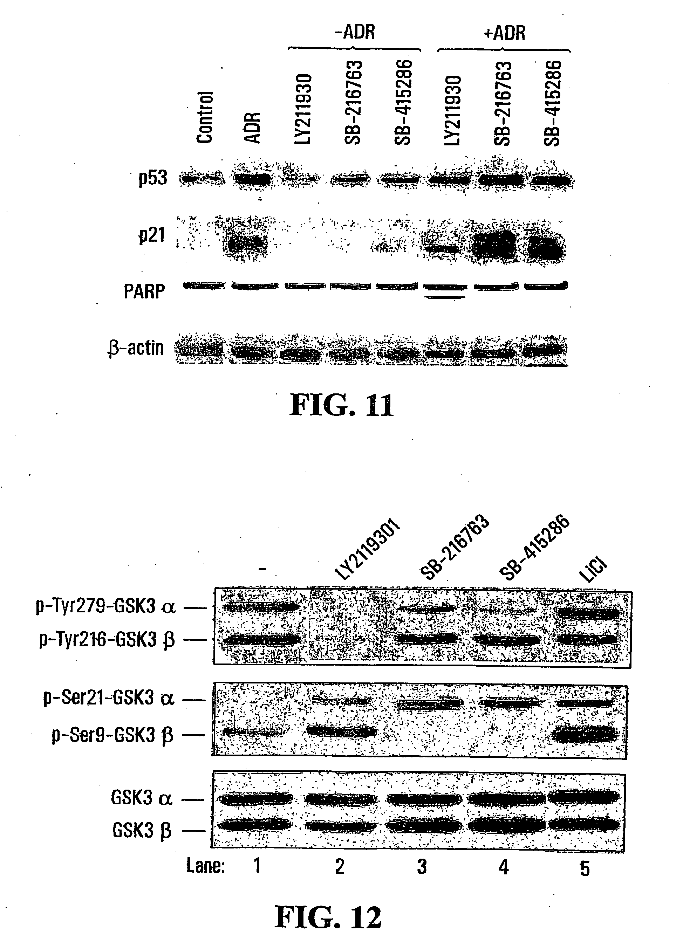

**Pharmacological modulation of GSK-3β but not GSK-3a promotes p53-dependent** apoptosis: To verify that GSK-3β modulation accounts for the above-observation, we examined the effects of three other structurally-unrelated small molecule GSK-3 inhibitors: SB-216763, SB-415-286, and lithium chloride (LiCl) (**Figure 8**). The former two compounds are competitive ATP inhibitors that are selective GSK-3 inhibitors (Coghlan, M. P., et al. Chem Biol, 7: 793-803, 2000), whereas lithium inhibits GSK-3β by acting as a competitive inhibitor of Mg²⁺, while having no inhibitory effect on other protein kinases (Watcharasit, P., et al., J Biol Chem, 278: 48872-48879, 2003; Ryves, W. J. and Harwood, A. J., Biochem Biophys Res Commun, 280: 720-725, 2001; Klein, P. S. and Melton, D. A., Proc Natl Acad Sci U S A, 93: 8455-8459, 1996). Similar to LY2119301, lithium treatment induced a strong apoptosis in HCT116 cells exposed to chemotherapeutic agents in a p53-dependent manner (Figure 9). Surprisingly, SB-126763 and SB-415286 had no effect on ADR-treated cells, even at high concentrations up to 20 µM (**Figure 10**). This is in contrast to LY2119301 which was able to induce apoptosis in ADR-treated cells at a concentration as low as 250 nM (Figure 10). To examine if SB-216763 and SB-415286 block p53 pathway in response to DNA damage, we performed immunoblot analysis and found that these two inhibitors had no affect on p53 stability or on p53-mediated p21 induction by DNA damage (**Figure 11**). Thus, the ability of LY2119301 to induce apoptosis in stressed cells appears to be closely correlated with its ability to inhibit p53-p21 pathway. After DNA damage, our results indicate that LY2119301 prevents the p53 stabilization and p21 induction, while other inhibitors had no effect on p53 action.

To investigate whether these compounds affect GSK-3 differentially, we performed immunoblot analysis. GSK-3 contains two isoforms (α and β) and their activities can be regulated by activating phosphorylation on Tyr216/Tyr279, or by inhibitory phosphorylation on Ser21/Ser9 (Cohen, P. and Frame, S., Nat Rev Mol Cell Biol, 2: 769-776, 2001). Specific GSK-3 inhibitors can either inhibit activating tyrosine phosphorylation or induce inhibitory serine autophosphorylation (Cohen, P. and Frame, S., Nat Rev Mol Cell Biol, 2: 769-776, 2001; Cohen, P. and Goedert, M., Nat Rev Drug Discov, 3: 479-487, 2004; Zhang, F., et al., J Biol Chem, 278: 33067-33077, 2003; Cole, A., et al., Biochem J, 377: 249-255, 2004). As such, phosphorylation status of these specific residues was used as surrogate marker for GSK-3α/β activity. Treatment of HCT116 cells with LY2119301 and lithium resulted in an identical increase in inhibitory serine phosphorylation on GSK-3β, and to a much lesser extent on GSK-3α (**Figure 12**, lanes 2&5). In contrast, SB-126763 and SB-415286 only induced serine 21 phosphorylation on GSK-3α and even slightly reduced the serine 9 phosphorylation on GSK-3β (**Figure 12**, lanes 3 & 4). Furthermore, LY2119301 almost completely blocked the active tyrosine phosphorylation on both GSK-3α and GSK-3β, while SB-126763 and SB-415286 reduced tyrosine phosphorylation on GSK-3α only (**Figure 12**, lanes 3 & 4). These findings suggest that LY2119301 and lithium inhibit both GSK-3α and GSK-3β, but predominately act on GSK-3β, whereas SB-126763 and SB-415286 were only inhibitory to GSK-3α in HCT116 cells, though both of them were previously shown to be equally effective at inhibiting GSK-3α and GSK-3β by using peptide-based *in vitro* protein kinase assays (Coghlan, M. P., et al., Chem Biol, 7: 793-803, 2000). Therefore, the apoptotic effect of GSK-3 inhibitors appears to be associated with the specific inactivation of GSK-3β, but not GSK-3α. Importantly, lithium, in contrast to LY2119301, did not block the activating tyrosine phosphorylation of GSK-3α/β (**Figure 12**, lane 5), but was still able to induce the apoptotic phenotype. Thus, inhibition of activating tyrosine phosphorylation is not required for the apoptotic effect of GSK-3β inhibitor. Instead, the induction of inhibitory serine 9 phosphorylation of GSK-3β is the common feature of both LY2119301 and lithium and thus appears to be associated with their apoptotic effects. The induction of serine 9 phosphorylation of GSK-3β by LY2119301 and lithium is not likely the result of activation of other upstream kinases such as AKT, since lithium is known to increase serine autophosphorylation through direct inhibition of GSK-3 itself, probably through inhibition of GSK-3-dependent phosphatase 1(Klein, F. S. and Melton, D. A., Proc Natl Acad Sci U S A, 93: 8455-8459, 1996). Furthermore, treatment of cells with LY294002 that inhibits AKT did not block the serine 9 phosphorylation triggered by LY2119301 and lithium (data not shown).

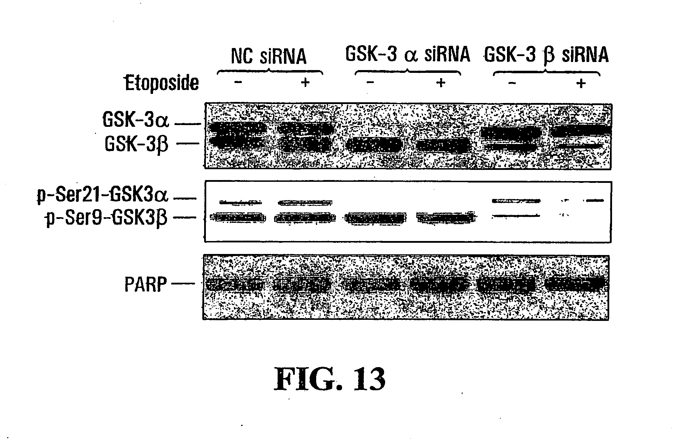

To test if GSK-3β knockdown by DNA interference would also sensitize DNA damage-induced apoptosis, HCT116 cells were transfected with ASK-3β, GSK-3α or negative control siRNA (NC siRNA)) for 48 h and followed by etoposide treatment. **Figure 13** shows that RNA interferences efficiently and specifically knocked down the expression of GSK-3α and GSK-3β, respectively. However, knockdown of GSK-3β or GSK-3α did not result in more cell death in response to etoposide, as no obvious PARP cleavage was observed. Thus, mere reduction of GSK-3β levels was not sufficient to trigger cell death in response to DNA damage. This observation is consistent with the notion that the induction of inhibitory serine 9 phosphorylation of GSK-3β might be important for the efficient apoptosis induction in colorectal cancer cells in response to DNA damage. GSK-3β siRNA, which could not induce serine 9 phosphorylation, failed to mimic the apoptotic effect of GSK-3β inhibitors.

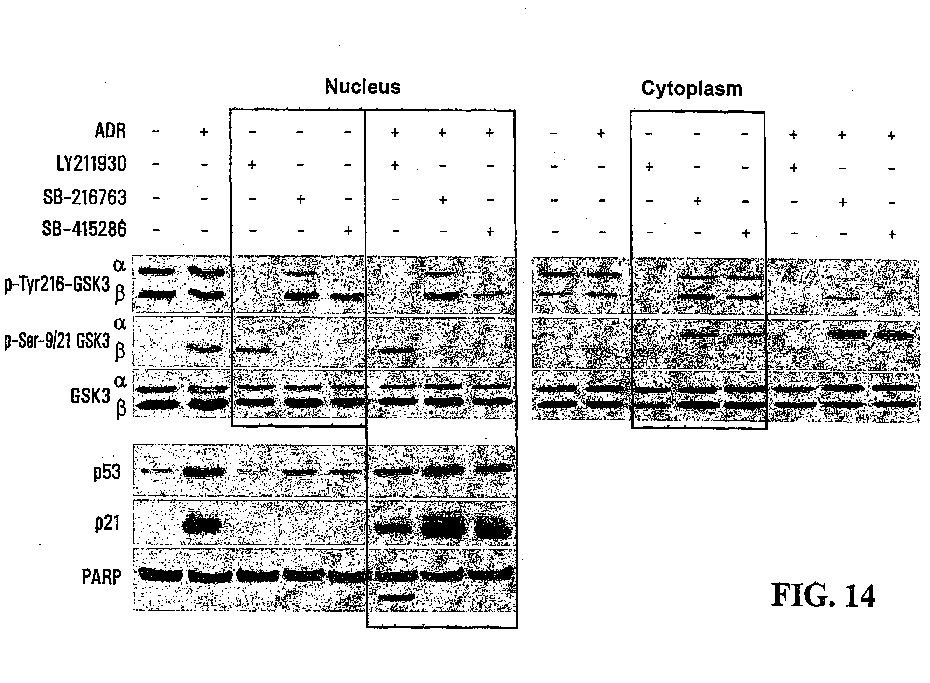

We next examined the mechanisms underling the different effects of GSK inhibitors on apoptosis and p53 function. Because GSK-3 is located in both cytosolic and nuclear compartments and GSK-3α/β is inhibited by phosphorylation of Ser-21(α) or Ser-9 (β) and activated by phosphorylation of Tyr- 279(a) or Tyr-216(β), we looked for changes in the phosphorylation state of GSK-3 after treatment with GSK inhibitors and DNA damage. After etoposide treatment, there were no changes in the levels of cytosolic and nuclear GSK-3α and GSK-3β and nor in their phosphorylation states, as evaluated by specific anti-Ser-9/21 or anti-Tyr-216 antibodies (**Figure 14**). Interestingly, LY2119301 triggered a serine phosphorylation on GSK-3β in both the cytoplasm and the nucleus and blocked tyrosine phosphorylation of both a and β isoforms. By contrast, the other two GSK inhibitors triggered serine phosphorylation on GSK-3α in the cytoplasm but not in the nucleus and had no effect on serine phosphorylation on GSK-3β. Consistently, SB-126763 and SB-415286 did not block the active tyrosine phosphorylation of GSK-3β in the nucleus. These data suggest that LY2119301 is highly selective on GSK-3β, whereas the other two inhibitors inhibit primarily on cytosolic GSK-3α, though both of them have been shown to inhibit GSK-3β as effectively as GSK-3a using an *in vitro* assay (Coghlan et al., 2000). The data suggest that nuclear GSK-3β but not GSK-3α regulates p53 function in response to DNA damage, which is highly associated with its ability to regulate cellular response to DNA damage. Of note, inhibition of nuclear GSK-3β is sufficient to reduce the basal level of p53 abundance, suggesting that GSK-3β regulates p53 accumulation in both normal and stressed cells.

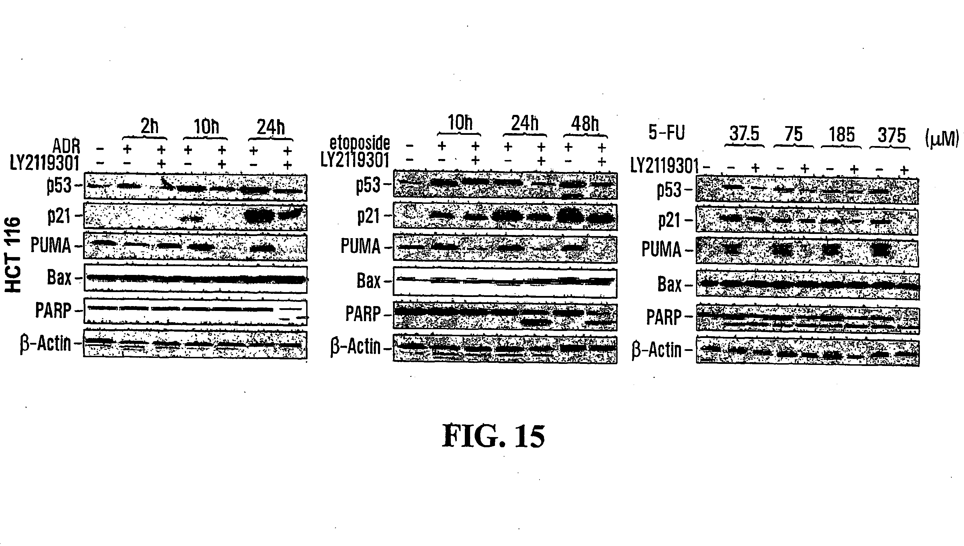

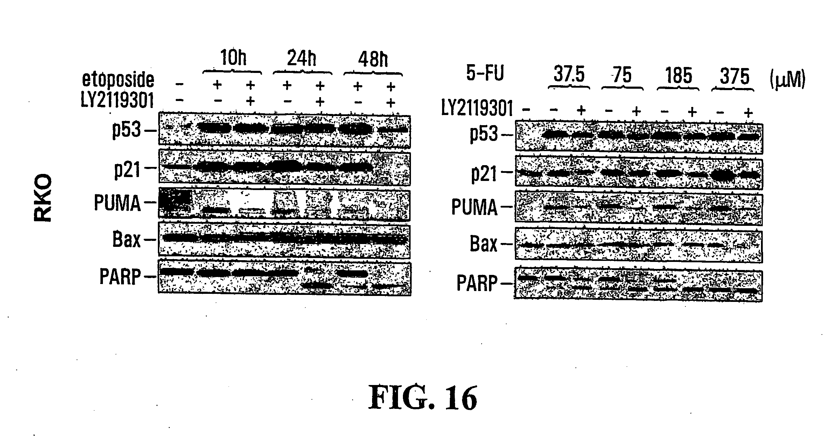

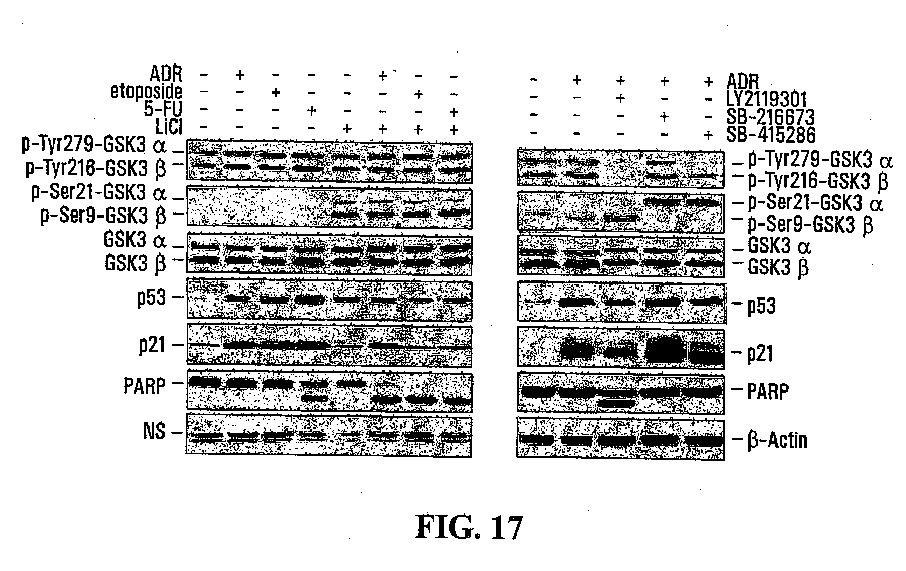

**Pharmacological modulation of GSK-3β impairs p53 accumulation and transactivation of p53 target genes in response to genotoxic stress:** To determine the effects of GSK-3β inhibitors on p53 response, we performed immunoblot analyses of p53 and p53 targets. HCT116 (**Figure 15**) and RKO (**Figure 16**) cells were treated with ADR, etoposide and 5-FU in the presence or absence of LY2119301. Cells treated with chemotherapeutic agents exhibited progressive accumulation of p53 and activation of its target genes such as p21 and Puma, mediating growth arrest and apoptosis, respectively. In the presence of LY2119301, p53 accumulation induced by chemotherapy was markedly and consistently impaired, as was induction of p21 and Puma. Thus, LY2119301 impaired p53 accumulation in response to genotoxic stress and reduced its transcriptional activity. Importantly, expression of Bax, a p53 target known to be essential for mitochondrial-mediated apoptosis in colon cancer (Zhang, L., et al., Science, 290: 989-992, 2000), was not induced following p53 activation. This is in agreement with previous reports that Bax is not a robust target of p53 in colon cancer cells (Yu, J., et al., Proc Natl Acad Sci U S A, 96: 14517-14522, 1999; Kokontis, J. M., et al., Oncogene, 20: 659-668, 2001). Accordingly, LY21193-01 treatment had no effect on Bax levels (**Figure 15** **and** **16**). Similarly, treatment with lithium that inhibits GSK-3β as well, also impaired p53 accumulation in response to ADR, etoposide and 5-FU, reduced the induction of p53 target gene p21 (**Figure 17**, left panel). Consistent with the apoptotic phenotype, cleavage of PARP was remarkably increased in cells treated with genotoxic agents in the presence of LY2119301 or lithium. In contrast, SB-126763 and SB-415286 that are only inhibitory to GSK-3α in these cells had no effect on p53 accumulation and p21 induction, and did not induce PARP cleavage (**Figure 17**, right panel).

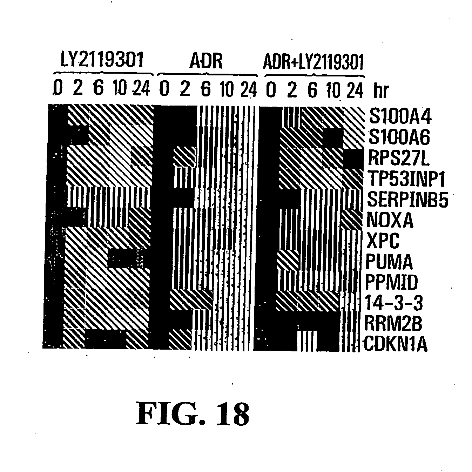

To extend these observations to other p53 targets, we performed global expression profiling of p53-induced genes using microarray analysis. We had previously identified a subset of p53 responsive genes in HCT116 cells (Kho, P. S., et al., J Biol Chem, 279: 21183-21192, 2004). Concurrent treatment with ADR and LY2119301 uniformly attenuated the induction of these genes (**Figure 18**), suggesting that GSK-3β plays a critical and global role in p53-dependent transactivation. The paradoxical observation that the GSK-3β inactivation promotes p53-mediated apoptosis, while abrogating transactivation of p53 targets, including Puma, raises an intriguing possibility that GSK-3β inhibitors promote p53-dependent apoptosis through a mechanism independent of p53-mediated transcription.

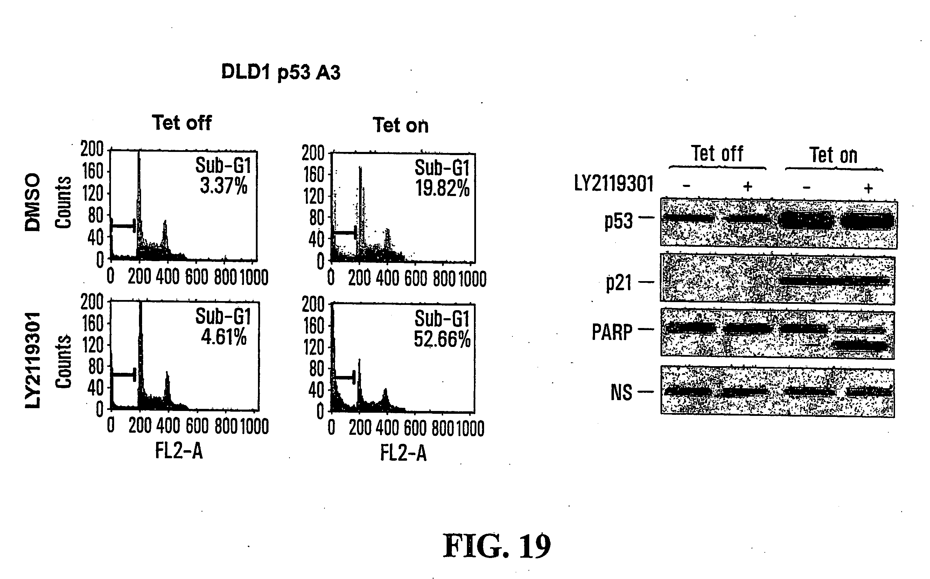

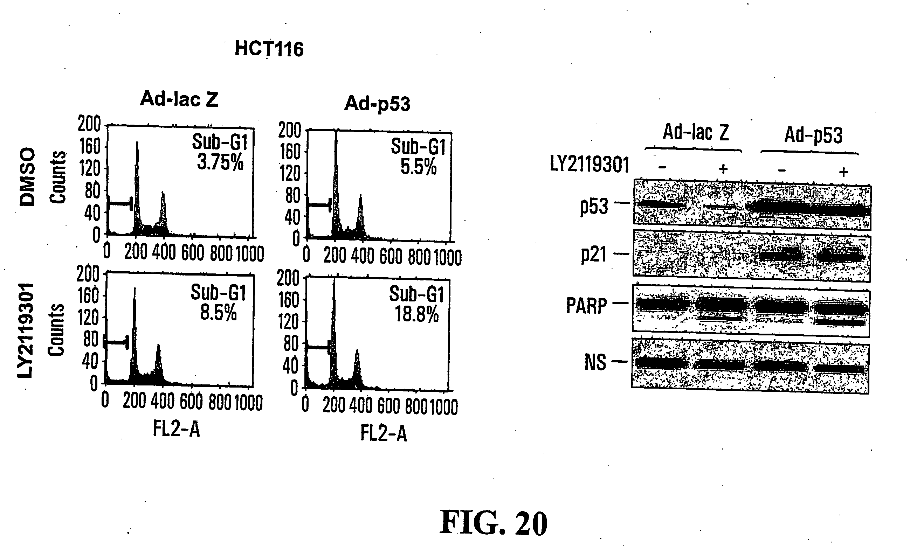

**GSK-3β inhibitor LY2119301 promotes apoptosis induced by exogenous p53:** We next investigated whether GSK-3β inactivation also promotes apoptosis induced by exogenous over-expression of p53. We used two p53 expressing systems: a tetracycline-inducible p53 expressing colon cancer DLD1 cell line (Yu, J., et al., Proc Natl Acad Sci U S A, 96: 14517-14522, 1999) and HCT116 cells infected with an adenovirus expression p53. In both cases, apoptosis induced by enforced p53 expression was significantly increased in the presence of LY2119301 (**Figures 19** and **20**). Under these circumstances, LY2119301 had no effect on ectopically expressed p53 protein levels, or p53 dependent transcription of p21 (**Figures 19** and **20**, right panels), probably due to non-physiological regulation of p53 in this context. Therefore, this induction of apoptosis was not likely due to the reduced expression of p21, since it has been reported that altered balance between p21 and p53 pro-apoptotic targets such as Puma can switch the p53 response from growth arrest to apoptosis (Seoane, J., et al., Nature, 419: 729-734, 2002; Iyer, N. G., et al., Proc Natl Acad Sci USA, 101: 7386-7391, 2004).

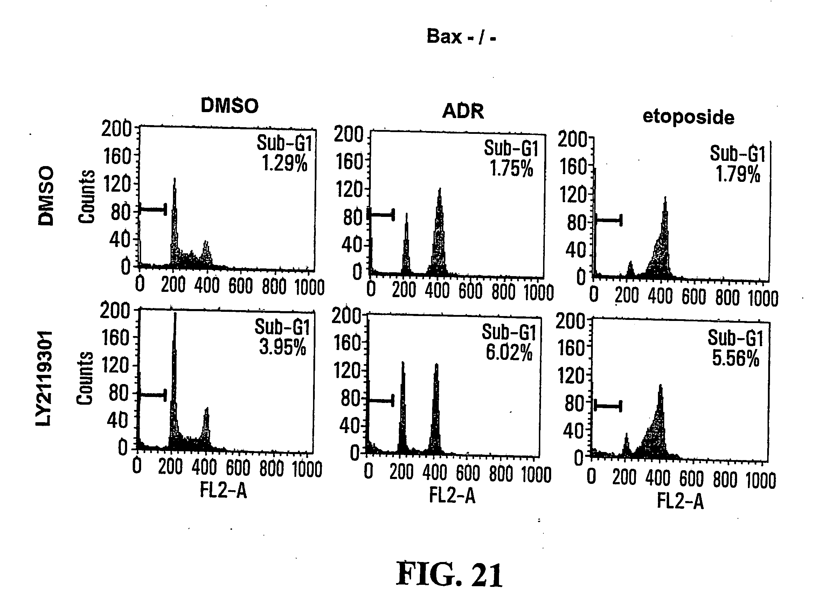

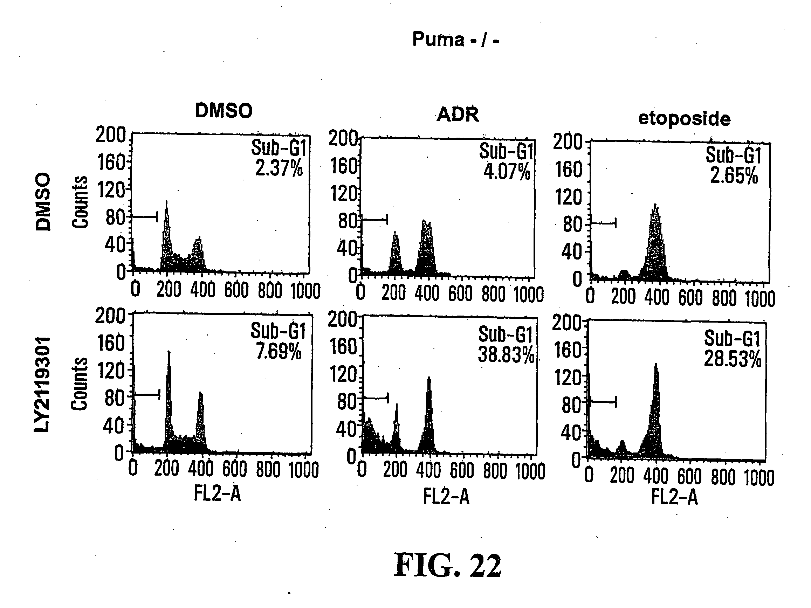

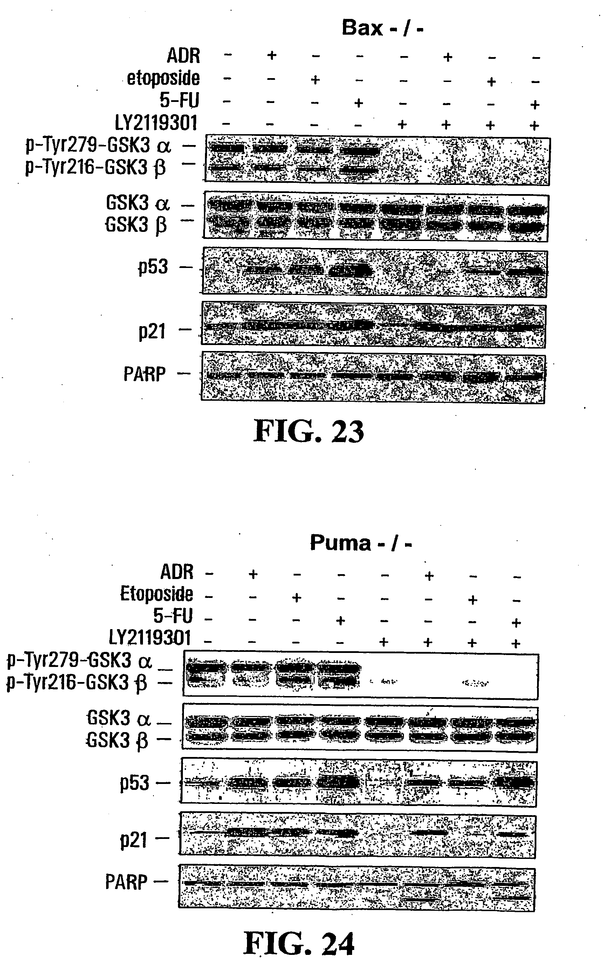

**Apoptosis promoted by GSK-3β inhibitor requires Bax but not Puma**: Proapoptotic Bcl2 family members Bax and Puma have been implicated in p53-mediaed apoptosis (Yu, J., et al., Mol Cell, 7: 673-682, 2001; Yu, J., et al., Proc Natl Acad Sci USA, 100: 1931-1936, 2003). To further asses the roles of Bax and Puma in p53-dependent apoptosis after GSK-3β inactivation, we used HCT116 cells with either of these genes deleted by homologous recombination (Zhang, L., et al., Science, 290: 989-992, 2000; Yu, J., et al., Proc Natl Acad Sci U S A, 100: 1931-1936, 2003). When co-treated with LY2119301 and ADR or etoposide, Bax ^{-/-}cells were completely refractory to apoptosis (**Figure 21**). In contrast, Puma ^{-/-} cells were still able to undergo marked apoptosis, identical to parental HCT116 cells (**Figure 22**). Consistently, PARP cleavage was not observed in Bax^{-/-} cells (**Figure 23**) but evident in Puma ^{-/-} cells (**Figure 24**), though in both cell lines p53 accumulation and induction of p21 after ADR or etoposide treatment was similarly reduced by LY2119301 (**Figures 23** and **24**). These results indicate that p53-dependent but transcription-independent apoptosis triggered by GSK-3β inhibitor proceeds through a pathway that requires Bax but not Puma. Supporting this notion, colon cancer LoVo cells were resistant to the apoptosis induced by LY2119301 upon DNA damage (data not shown). LoVo cells are p53 wild-type but do not express Bax protein due to a frame-shift mutation in the Bax gene (Ionov, Y., et al., Proc Natl Cad Sci U S A, 97: 10872-10877,2000).

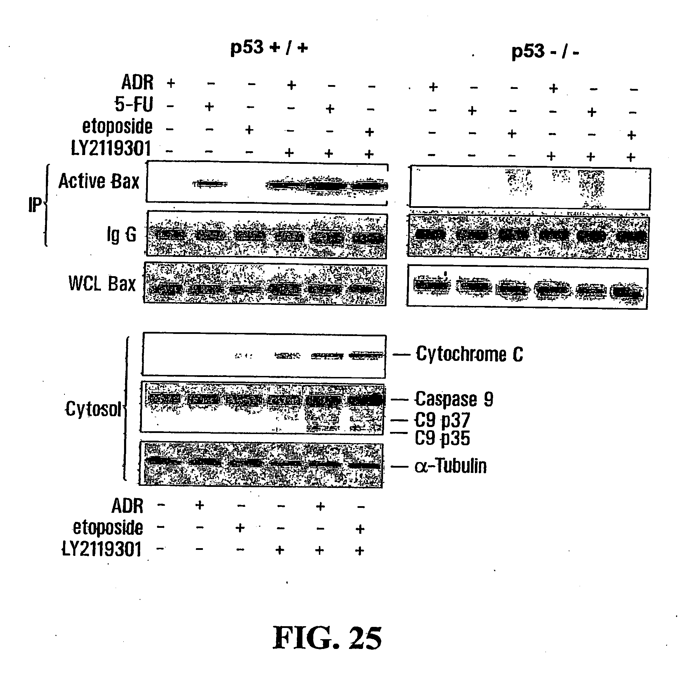

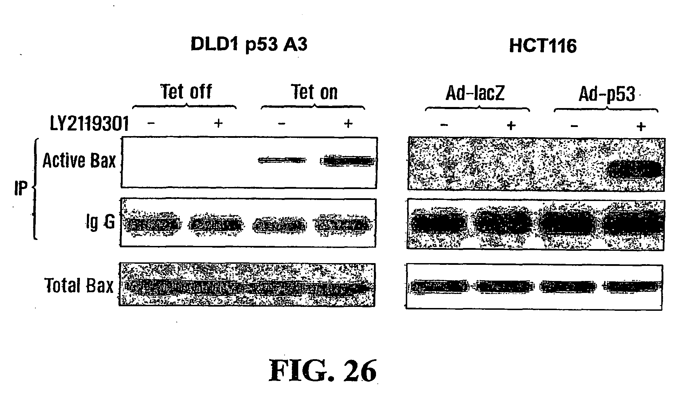

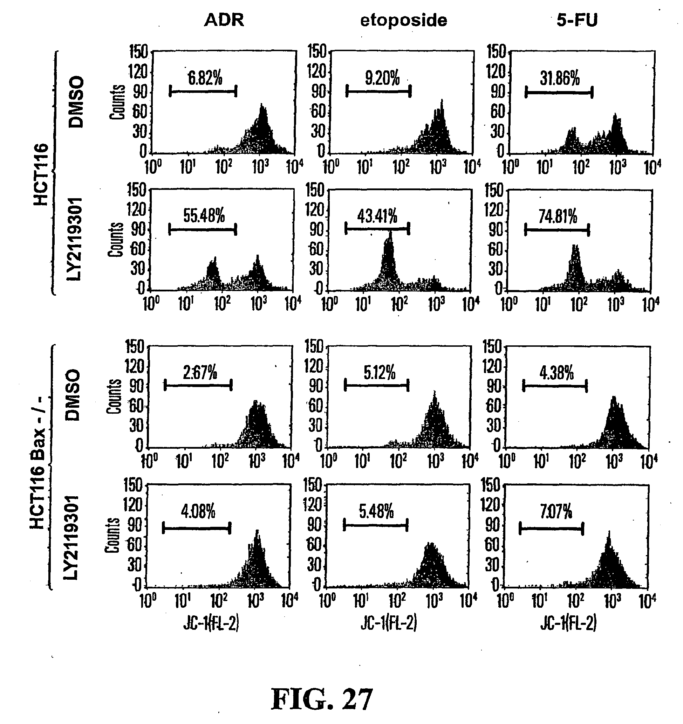

**GSK-3β inhibition promotes p53-dependent conformational activation of Bax and induces mitochondrial cell death:** Bax is known to undergo a conformational change during apoptosis (Wolter, K. G., et al., J Cell Biol, 139: 1281-1292, 1997). We next examined whether GSK-3β inhibitor induces a conformational activation of Bax after DNA damage. Co-treatment of HCT116 cells with LY2119301 and genotoxic agents ADR, etoposide and 5-FU dramatically increased levels of conformationally active Bax (**Figure 25**), which can be detected by a specific anti-Bax monoclonal antibody 6A7, as previously described (Suzuki, M., et al., Cell, 103: 645-654, 2000; Nechushtan, A., et al., Embo J, 18: 2330-2341, 1999). Bax activation appears to be p53-dependent, as there was no detectable change in Bax conformation in HCT116 p53 -/- cells under the same conditions (**Figure 25**). Consistently, increased cytochrome c release and caspase 9 processing were evident after DNA damage in the presence of LY2119301 (**Figure 25**, lower panel). Bax activation was similarly seen in DLD1 cells and HCT116 tells with concurrent p53 over-expression in the presence of LY2119301 (**Figure 26**). Moreover, a significant reduction of mitochondrial membrane potential, as determined by JC-1 staining and FACS, was detected in Bax ^{+/+} cells -co-treated with LY2119301 and other genotoxic treatments, but not in Bax^{-/-} cells under the same conditions (**Figure 27**). Taken together, these findings demonstrate that GSK-3β inhibitor promotes p53-dependent apoptosis through a Bax-mediated mitochondrial pathway.

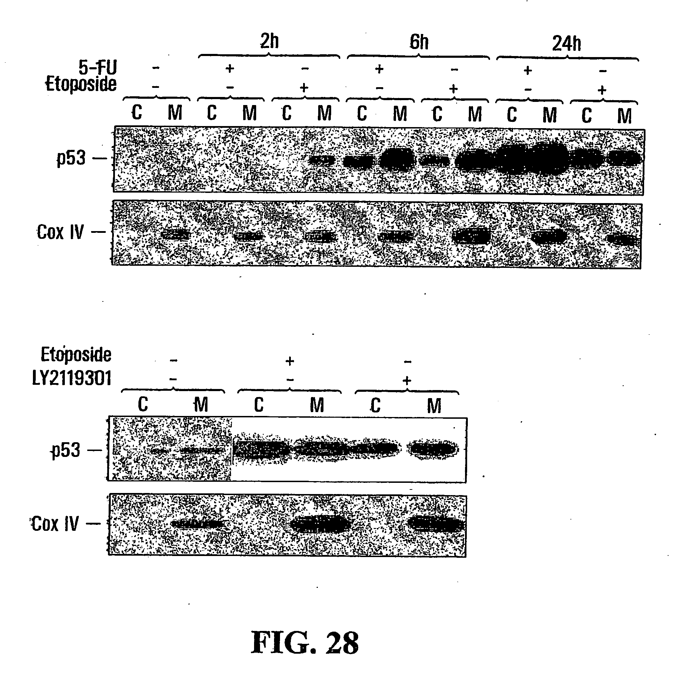

The above results raise the possibility that GSK-3β inhibitors might enable the p53 accumulation in the mitochondria, thus accounting for the observed apoptosis following DNA damage. To address this issue, we first monitored the p53 accumulation in both cytosol and mitochondria at different time points following etoposide and 5-FU treatments. We found that p53 was accumulated in the mitochondria as early as 2 hours after etoposide or 5-FU treatment, while the accumulation of p53 in the cytosol was not seen until 6 hours after the drug treatments (**Figure 28**). This is consistent with a recent report showing that DNA damage triggers a rapid p53 mitochondrial accumulation in mouse thymus, which led to the first wave of cell death (Erster, S., et al., Mol Cell Biol, 24: 6728-6741, 2004). However, etoposide-induced p53 accumulation in the mitochondria in HCT116 cells failed to induce apoptosis. This demonstrates that p53 accumulation in mitochondria per se is not sufficient to activate apoptosis in HCT116 cells. Furthermore, in the presence of LY2119301, etoposide-induced p53 accumulations were similarly decreased in both cytosol and mitochondria (**Figure 28**), although a robust apoptosis was induced under these conditions. Thus, we conclude that the mitochondrial p53 levels in colorectal cancer cells are likely not correlated with the levels of apoptosis induction in colorectal cancer cells, and that the apoptotic effects of GSK-3β inhibitors on p53 response is likely not due to increased translocation of p53 to the mitochondria.

As can be understood by one skilled in the art, many modifications to the exemplary embodiments described herein are possible. The invention, rather, is
defined by the claims.

## Claims

1. An agent that inhibits GSK-3β activity for use in the treatment of a cellular proliferative disorder of a colorectal cancer cell in a subject currently undergoing chemotherapy with a DNA damaging agent, wherein inhibition of GSK-3β activity causes an increase in inhibitory serine phosphorylation of GSK-3β and wherein the colorectal cancer cell is capable of being induced to undergo p53-dependent apoptosis.

2. The agent for use of claim 1, wherein the use comprises inducing cell death in the colorectal cancer cell.

3. The agent for use of claim 1 or 2, wherein the subject is a human.

4. The agent for use of any one of the preceding claims, wherein DNA damaging agent is Adriamycin, 5-fluorouracil, etoposide or camptothecin.

5. The agent for use of any one of the preceding claims, wherein said agent that inhibits GSK-3β activity is a lithium-containing compound.

6. The agent for use of any one of the preceding claims, wherein the agent that inhibits GSK-3β activity is LY2119301 or lithium chloride.

7. A method of inducing cell death *in vitro* in a colorectal cancer cell currently undergoing chemotherapy with a DNA damaging agent comprising contacting the cell with an agent that inhibits glycogen synthase kinase-3β activity, wherein inhibition of GSK-3β activity causes an increase in inhibitory serine phosphorylation of GSK-3β and wherein the colorectal cancer cell is capable of being induced to undergo p53-dependent apoptosis.

8. The method of claim 7, wherein said inhibiting induces p53-dependent apoptosis.

9. The method of claim 7 or 8, wherein the chemotherapy induces p53-dependent cell cycle arrest.

10. The method of any one of claims 7 to 9, wherein said inhibiting inhibits p53-activated transcription.

11. The method of any one of claims 7 to 10, wherein the chemotherapy involves Adriamycin, 5-fluorouracil, etoposide or camptothecin.

12. The method of any one of claims 7 to 11, wherein the agent that inhibits GSK-3β activity is a lithium-containing compound.

13. The method of any one of claims 7 to 11, wherein the agent that inhibits GSK-3β activity is lithium chloride.

14. The method of any one of claims 7 to 11, wherein the agent that inhibits GSK-3β activity is LY2119301.

15. A method of identifying a compound useful for inducing cell death in a cell, comprising:
(a) contacting glycogen synthase kinase-3β with a test compound; and
(b) determining whether inhibitory serine phosphorylation of said glycogen synthase kinase-3β is increased in the presence of said test compound, thereby inhibiting the activity of said glycogen synthase kinase-3β.

16. The method according to claim 15, wherein step (a) comprises contacting a cell expressing said glycogen synthase kinases-3β with said test compound.

## Patentansprüche

1. Ein Mittel, welches die GSK-3β Aktivität hemmt zur Anwendung in der Behandlung einer Zellproliferationserkrankung einer Colorectal Krebszelle in einem Subjekt welches gerade eine Chemotherapie mit einem DNA schädigenden Mittel erhält, wobei die Hemmung der GSK-3β Aktivität zu einem Anstieg an hemmender Serin Phosphorylierung von GSK-3β führt und wobei in der Colorectal Krebszelle eine p-53 abhängige Apoptosis induziert werden kann.

2. Das Mittel zur Anwendung gemäss Anspruch 1, wobei die Anwendung das Induzieren des Zelltods in der Colorectal Krebszelle umfasst.

3. Das Mittel zur Anwendung gemäss Anspruch 1 oder 2, wobei das Subjekt ein Mensch ist.

4. Das Mittel zur Anwendung gemäss einem der vorhergehenden Ansprüche, wobei das DNA schädigende Mittel Adriamycin, 5-Fluorouracil, Etoposid oder Camptothecin ist.

5. Das Mittel zur Anwendung gemäss einem der vorhergehenden Ansprüche, wobei das Mittel, das die GSK-3β Aktivität hemmt, eine Lithium enthaltende Verbindung ist.

6. Das Mittel zur Anwendung gemäss einem der vorhergehenden Ansprüche, wobei das Mittel, das die GSK-3β Aktivität hemmt, LY2119301 oder Lithiumchlorid ist.

7. Ein Verfahren zur in vitro Zelltod Induzierung in einer Colorectal Krebszelle, die gerade einer Chemotherapie mit einem DNA schädigenden Mittel ausgesetzt ist, wobei das Verfahren umfasst: das in Kontakt bringen der Zelle mit einem Mittel, welches die Glycogen Synthase Kinase 3β Aktivität hemmt, wobei die Hemmung der GSK-3β Aktivität zu einem Anstieg an hemmender Serin Phosphorylierung von GSK-3β führt und wobei in der Colorectal Krebszelle eine p-53 abhängige Apoptosis induziert werden kann.

8. Das Verfahren gemäss Anspruch 7, wobei besagte Hemmung p-53 abhängige Apoptosis induziert.

9. Das Verfahren gemäss Anspruch 7 oder 8, wobei die Chemotherapie p53 abhängige Zellzyklus Aussetzung induziert.

10. Das Verfahren gemäss einem der Ansprüche 7 bis 9, wobei besagte Hemmung die p53 aktivierte Transkription hemmt.

11. Das Verfahren gemäss einem der Ansprüche 7 bis 10, wobei die Chemotherapie Adriamycin, 5-Fluorouracil, Etoposid oder Camptothecin beinhaltet.

12. Das Verfahren gemäss einem der Ansprüche 7 bis 11, wobei das Mittel, das die GSK-3β Aktivität hemmt, eine Lithium enthaltende Verbindung ist.

13. Das Verfahren gemäss einem der Ansprüche 7 bis 11, wobei das Mittel, das die GSK-3β Aktivität hemmt, Lithiumchlorid ist.

14. Das Verfahren gemäss einem der Ansprüche 7 bis 11, wobei das Mittel, dass die GSK-3β Aktivität hemmt, LY2119301 ist.

15. Ein Verfahren zur Identifizierung einer Verbindung, die zur Zelltod Induzierung in einer Zelle verwendbar ist umfassend:
(a) in Kontakt bringen von Glycogen Synthase Kinase-3β mit einer Test Verbindung und
(b) Bestimmen ob die hemmende Serin Phosphorylierung von besagter Glycogen Synthase Kinase-3β erhöht wird in der Anwesenheit von besagter Testverbindung dabei die Aktivität von besagter Glycogen Synthase Kinase-3β hemmend.

16. Ein Verfahren gemäss Anspruch 15, wobei Stufe (a) das In Kontakt Bringen einer Zelle, die besagte Glycogen Synthase Kinase-3β exprimiert, mit besagter Testverbindung umfasst.

## Revendications

1. Un agent qui inhibe l'activité de GSK-3β pour une utilisation dans le traitement d'un trouble prolifératif cellulaire d'une cellule du cancer colorectal chez un sujet en cours de traitement par chimiothérapie avec un agent endommageant l'ADN, dans lequel l'inhibition de l'activité de la GSK-3β provoque une augmentation de la phosphorylation de sérine inhibitrice de la GSK-3β, et dans lequel la cellule du cancer colorectal est susceptible d'être amenée à subir une apoptose p53-dépendante.

2. L'agent pour son utilisation selon la revendication 1, dans lequel l'utilisation comprend l'induction de la mort cellulaire dans la cellule du cancer colorectal.

3. L'agent pour son utilisation selon la revendication 1 ou 2, dans lequel le sujet est un humain.

4. L'agent pour son utilisation selon l'une quelconque des revendications précédentes, dans lequel l'agent endommageant l'ADN est l'adriamycine, le 5-fluorouracile, l'étoposide ou camptothécine.

5. L'agent pour son utilisation selon l'une quelconque des revendications précédentes, dans lequel ledit agent qui inhibe l'activité de GSK-3β est un composé contenant du lithium.

6. L'agent pour son utilisation selon l'une quelconque des revendications précédentes, dans lequel l'agent qui inhibe l'activité de GSK-3β est LY2119301 ou le chlorure de lithium.

7. Une méthode *in vitro* d'induction de la mort cellulaire dans une cellule du cancer colorectal actuellement l'objet d'une chimiothérapie avec un agent endommageant l'ADN comprenant le contact de la cellule avec un agent qui inhibe l'activité de la glycogène synthase kinase-3β, dans laquelle l'inhibition de l'activité de la GSK-3β provoque une augmentation de la phosphorylation de sérine inhibitrice de la GSK-3β, et dans laquelle la cellule du cancer colorectal est susceptible d'être amenée à subir une apoptose p53-dépendante.

8. La méthode selon la revendication 7, dans laquelle ledit inhibiteur induit l'apoptose p53-dépendante.

9. La méthode selon la revendication 7 ou 8, dans laquelle la chimiothérapie induit l'arrêt du cycle cellulaire p53-dépendant.

10. La méthode selon l'une quelconque des revendications 7 à 9, dans laquelle ledit inhibiteur inhibe la transcription p53-activée.

11. La méthode selon l'une quelconque des revendications 7 à 10, dans laquelle la chimiothérapie comprend l'adriamycine, le 5-fluorouracile, l'étoposide ou camptothécine.

12. La méthode selon l'une quelconque des revendications 7 à 11, dans laquelle l'agent qui inhibe l'activité de GSK-3β est un composé contenant du lithium.

13. La méthode selon l'une quelconque des revendications 7 à 11, dans laquelle l'agent qui inhibe l'activité de GSK-3β est le chlorure de lithium.

14. La méthode selon l'une quelconque des revendications 7 à 11, dans lequel l'agent qui inhibe l'activité de GSK-3β est LY2119301.

15. Une méthode d'identification d'un composé utile pour induire la mort cellulaire dans une cellule, comprenant:
(a) contacter la glycogène synthase kinase-3β avec un composé à tester, et
(b) déterminer si l'inhibition de la phosphorylation de sérine de ladite glycogène synthase kinase-3β est augmentée en présence dudit composé à tester, ainsi inhibant l'activité de ladite glycogène synthase kinase-3β.

16. La méthode selon la revendication 15, dans lequel l'étape (a) comprend la mise en contact d'une cellule exprimant ladite glycogène synthase kinase 3β avec ledit composé à tester.
